# EUROPÄISCHE PATENTANMELDUNG

(11) **EP 2 687 210 A1**
(43) Veröffentlichungstag der Anmeldung: **22.01.2014**
(21) Anmeldenummer: 13179993.4
(22) Anmeldetag: 21.06.2011
(51) Int. Cl.: A61K 31/198, A61K 31/426, A61K 31/427, A61K 31/4439, A61K 31/506, A61K 31/519, A61K 31/5377, A61P 7/06, A61K 45/06

(54) **Verwendung von Stimulatoren und Aktivatoren der löslichen Guanylatzyklase zur Behandlung von Sichelzellanämie und Konservierung von Blutersatzstoffen**

(30) Priorität: 25.06.2010 EP 10006623
(62) Teilanmeldung aus: 11726444.0
(71) Anmelder: Bayer Intellectual Property GmbH, 40789 Monheim am Rhein (DE)
(72) Erfinder: Stasch, Johannes-Peter, 42651 Solingen (DE); Becker-Pelster, Eva Maria, 42327 Wuppertal (DE); Trübel, Hubert, 42281 Wuppertal (DE); Himmel, Herbert, 45128 Essen (DE)
(74) Vertreter: BIP Patents

(57) **Zusammenfassung**

Die vorliegende Erfindung betrifft die neue Verwendung von Stimulatoren und Aktivatoren der löslichen Guanylatzyklase zur Behandlung von Sichelzellanämie und Konservierung von Blutersatzstoffen. Desweiteren betrifft die vorliegende Erfindung die Verwendung von Stimulatoren und Aktivatoren der löslichen Guanylatzyklase in Kombination mit PDE 5 Inhibitoren zur Behandlung von Sichelzellanämie und Konservierung von Blutersatzstoffen.

## Beschreibung

Die vorliegende Erfindung betrifft die neue Verwendung von Stimulatoren und/oder Aktivatoren der löslichen Guanylatzyklase zur Behandlung von Sichelzellanämie und Konservierung von Blutersatzstoffen. Des Weiteren betrifft die vorliegende Erfindung die Verwendung von Stimulatoren und/oder Aktivatoren der löslichen Guanylatzyklase in Kombination mit PDE5 Inhibitoren zur Behandlung von Sichelzellanämie und Konservierung von Blutersatzstoffen.

In den letzten Jahrzehnten mehrt sich das Wissen bezüglich der Rolle von NO in der Blutfluss-Regulation und der kardiovaskulären Hömostase. Pulmonare Hypertension (PH) ist eine häufige Komplikation bei der Sichelzellerkrankung, der eine Störung in der NO-Signalkaskade in Kombination mit erhöhtem oxidativem Stress zu Grunde liegt. PH ist dabei eine Komplikation bei Sichelzellanämie und ein starker Prädiktor für Mortalität. Bei Sichelzellpatienten findet sich eine Resistenz gegenüber NO, hevorgerufen zum einen durch das unverzügliche Abfangen von gebildetem NO durch freies Hämoglobin (Hb), welches beim Zerfall der Erythrocyten freigestzt wird, zum anderen aber auch durch eine erhöhte Oxidation und Degradation der löslichen Guanylatzyklase (sGC), die durch NO als endogenen Liganden aktiviert wird. Zur Klärung der weiteren Pathophysiologie könnten insbesondere transgene Mäuse (exklusive Expression von humanem Sichelzellhämoglobin), die verschiedene Krankheitserscheinungen aufweisen (u.a. PH), hilfreich sein (Blood. 2007;109: 3088-3098).

Hier beanspruchen wir die Anwendung von sGC-Aktivatoren und -Stimulatoren für die Behandlung von Sichelzellanämie und der damit verbundenen PH sowie anderen Krankheitserscheinungen (z.B. Endorganschäden, die Hirn, Niere oder Herz betreffen).

Daneben treten die gleichen o.g. pathophysiologischen Mechanismen in Kraft, wenn Bluttansfusionen (z.B. durch Lagerung etc. mit erhöhter freier Hb Konzentration) bei Patienten mit einer Transfusions-Indikation appliziert werden.

Weiterhin könnte die Kombination eines sGC-Aktivators und -Stimulators mit einem künstlichen Hb-basierten Sauerstoffträgers zukünftig die bisher beobachteten Nebenwirkungen [Weiskopf, Anaesthesia & Analgesia, 110:3; 659-661, 2010], die durch eine verminderte NO-Verfügbarkeit bedingt sind, mitigieren und somit eine klinische Anwendung möglich machen.

Durch sGC-Aktivatoren und Stimulatoren wird die lösliche Guanylatzyklase direkt stimuliert, sodass die fehlende NO-Wirkung ersetzt wird. sGC Stimulatoren werden nicht von freiem Hb beeinflusst.

Durch sGC Aktivatoren ist es möglich, auch oxidierte Formen der löslichen Guanylatzyklase unabhängig von NO direkt zu stimulieren. Diese oxidierte Form könnte sich in Geweben, die oxidativem Stress ausgesetzt sind, in höheren Konzentrationen anreichern, so dass es durch den Einsatz von sGC Aktivatoren auch zu einer gerichteten Behandlung von Geweben, die unter oxidativem Stress stehen, kommen sollte.

Deswegen sollten sGC Stimulatoren und sGC Aktivatoren unter akuten und insbesondere unter chronischem Einsatz positive Effekte auf den Blutdruck, die Sauerstoffsättigung und die resultierenden Plasma und Gewebs cGMP Spiegel unter experimentelle Bedingungen zeigen. Diese experimentellen Bedingungen bestehen zum einen aus gesunden Tieren, Tieren unter externer freier Hb Applikation oder auch gentechnisch veränderte Tiere wie z.B. Sichelzellmäuse (transgene Mäuse, die exklusiv humanes Sichelzell Hämoglobin beta exprimieren). Hierbei werden die Experimente mit sGC Stimulatoren und Aktivatoren "head to head" gegen den PDE5 Inhibitor Sildenafil durchgeführt, da Sildenafil kürzlich in der klinischen Erprobung (Phase II) bei Patienten mit Sichelzellanämie gescheitert ist. Es führte zu einer Erhöhung der Schmerzkrisen bei den Patienten, sodass die Studie vorzeitig abgebrochen wurde. Daher ist es zwingend notwendig, die Frage zu klären, ob es durch die Erhöhung von cGMP (im Falle von sGC Stimulatoren und Aktivatoren über eine Stimulation der sGC, im Falle von Sildenafil über eine Inhibition des Abbaus des gebildeten cGMP durch Hemmung der Phosphodiesterase 5) zu Stande kommt, oder ob dies eine Eigenschaft von Sildenafil oder allgemein der PDE5 Inhibitoren ist. Das bisherige klinische Programm mit sGC Stimulatoren und Aktivatoren zeigt bisher keine Hinweise, auf eine erhöhte Inzidenz von Schmerzepisoden bei den behandelten Probanden. Im Gegensatz dazu, wird bei PDE5 Inhibitoren wie Sildenafil, Vardenafil und Tadalafil immer wieder auf ein erhöhtes Vorkommen von Rückenschmerz hingewiesen. Deswegen könnte es von aussergewöhnlichem Interesse sein, o.g. sGC Stimulatoren und Aktivatoren bereits auf präklinischer Ebene von PDE 5 Inhibitoren differenziert werden können.

Basierend auf diesem Stand der Technik ist die Aufgabe der vorliegenden Erfindund die Bereitstellung von Substanzen zur Behandlung von Sichezellanämie und Konservierung von Blutersatzstoffen. Die Lösung dieser Aufgabe ist das Auffinden der im folgenden genannten sGC Stimulatoren und/oder sGC Aktivatoren alleine oder in Kombination sowie die Kombination von sGC Stimulatoren und/oder sGC Aktivatoren mit PDE5 Inhibitoren.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, an das Eisen-Zentralatom des Häms zu binden, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion, der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Arteriosklerose, Angina pectoris, Herzinsuffizienz, Myokardinfarkt, Thrombosen, Schlaganfall und sexueller Dysfunktion führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriff am Eisen-Zentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxymethyl-2'-furyl)-1-benzylindazol [YC-1; Wu et al., Blood 84 (1994), 4226; Mülsch et al., Brit. J. Pharmacol. 120 (1997), 681], Fettsäuren [Goldberg et al., J. Biol. Chem. 252 (1977), 1279], Diphenyliodonium-hexafluorophosphat [Pettibone et al., Eur. J. Pharmacol. 116 (1985), 307], Isoliquiritigenin [Yu et al., Brit. J. Pharmacol. 114 (1995), 1587] sowie verschiedene substituierte Pyrazol-Derivate (WO 98/16223) und hier als sGC Stimulatoren im Sinne dieser Erfindung zu verstehen sind.

Gegenstand der vorliegen Erfindung ist die Verwendung von Stimulatoren und/oder Aktivatoren der löslichen Guanylatzyklase zur Behandlung von Sichelzellanmämie und Konservierung von Blutersatzstoffen.

Besonders bevorzugt sind die Verbindungen (1)-(26) wie im folgenden dargsetellt:
- 2-[1-(2-Fluorbenzyl)-1H-pyrazolo[3,4-b]pyridin-3-yl]-5-(4-morpholinyl)-4,6-pyrimidindiamin (1), offenbart als Beispiel 16 in WO 00/06569,
- 2-[1-(2-Fluorobenzyl)-1H-pyrazolo[3,4-b]pyridin-3-yl]-5-(4-pyridinyl)-4-pyrimidinamin (2), offenbart als Beispiel 1 in WO 02/42301,
- Methyl-4,6-diamino-2-[1-(2-fluorobenzyl)-1H-pyrazolo[3,4-b]pyridin-3-yl]-5-pyrimidinyl-(methyl)carbamat (3), offenbart als Beispiel 8 in WO 03/095451,
- Methyl-4,6-diamino-2-[1-(2-fluorobenzyl)-1H-pyrazolo[3,4-b]pyridin-3-yl]-5-pyrimidinyl-carbamat (4), offenbart als Beispiel 5 in WO 03/095451
- 4-({(4-carboxybutyl)[2-(2-{[4-(2-phenylethyl)benzyl]oxy}phenyl)ethyl]amino} methyl) carbonsäure (5), offenbart als Beispiel 8a in WO 01/019780,
- Methyl-{4,6-diamino-2-[5-fluor-1-(2-fluorbenzyl)-1H-pyrazolo[3,4-b]pyridin-3-yl]pyrimidin-5-yl}carbamat (6),
- Methyl-{4,6-diamino-2-[5-fluor-1-(2-fluorbenzyl)-1H-pyrazolo[3,4-b]pyridin-3-yl]pyrimidin-5-yl}methylcarbamat (7),
- Methyl-{4,6-diamino-2-[5-fluor-1-(2-fluorbenzyl)-1H-pyrazolo[3,4-b]pyridin-3-yl]pyrimidin-5-yl}(2,2,2-trifluorethyl)carbamat (8),
- 5-Chlor-2-(5-chlorothiophen-2-sulfonylamino-N-(4-(morpholin-4-sulfonyl)-phenyl)-benzamid als Natriumsalz (9), offenbart in WO00/02851,
- 2-(4-Chloro-phenylsulfonylamino)-4,5-dimethoxy-N-(4-(thiomorpholin-4-sulfonyl)-phenyl)-benzamid (10), offenbart in WO00/02851,
- 1-{6-[5-Chloro-2-({4-trans-4-}trifluoromethyl)cyclohexyl]benzyl}oxy)phenyl]pyridin-2-yl}-5-(trifluoromethyl)-1H-pyrazol-4-carbonsäure (11), offenbart in WO 2009/032249,
- 1-[6-(2-(2-Methyl-4-(4-trifluoromethoxyphenyl)benzyloxy)-phenyl)pyridin-2-yl]-5-trifluoromethyl-pyrazol-4-carbonsäure (12), offenbart in WO 2009/071504,
- 1[6-(3,4-dichlorophenyl)-2-pyridinyl-5-(trifluoromethyl)-1H-pyrazole-4-cabonsäure (13), offenbart in WO 2009/068652,
- 1-({2-[3-Chlor-5-(trifluoromethyl)phenyl]-5-methyl-1,3-thiazol-4-yl}methyl)-1H-pyrazole-4-carbonsäure (14), 4-({2-[3-(Trifluoromethyl)phenyl]-1,3-thiazol-4-yl}methyl)benzoesäure (15) and 1-({2-[2-Fluoro-3-(trifluoromethyl)phenyl]-5-methyl-1,3-thiazol-4-yl}methyl)-1H-pyrazol-4-carbonsäure (16) offenbart in WO 2009/123316,
- 4-Amino-2-[5-chlor-3(3,3,3-triflourpropyl)-1H-indazol-1yl]-5,5-dimethyl-5,7-dihydro-6H-pyrrolo[2,3-d]pyrimidin-6-on (17), 4-Amino-2[5-chlor-3-(2,3,6-trifluorbenzyl)-1H-indazol-lyl]-5,5-dimethyl-5,7-dihydro-6H-pyrrolo[2,3-d]pyrimidin-6-on (18), 4-Amino-5,5-dimethyl-2-[3-(2,3,6-trifluorbenzyl)1H-thieno[3,4-c]pyrazol-1-yl]-5,7-dihydro-6H-pyrrolo[2,3-d]pyrimidin-6-on (19), 4-Amino-5,5-dimethyl-2-[3-(2,3,6-trifluorbenzyl)1H-thieno[2,3-d]pyrazol-1-yl]-5,5-dimethyl-5,7-dihydro-6H-pyrrolo[2,3-d]pyrimidin-6-on (20), 4-Amino-5,5-dimethyl-2-[7-(2,3,6-trifluorobenzyl)imidazo[1,5-b]pyridazin-5-yl]-5,7-dihydro-6H-pyrrolo[2,3-d]pyrimidin-6-on (21), 4-Amino-2-[6-chlor-3-(2,3,6-trifluorobenzyl)imidazo[1,5-a]pyridin-1-yl]]-5,5-dimethyl-5,7-dihydro-6H-pyrrolo[2,3-d]pyrimidin-6-on (22), 4-Amino-2-[6-fluor-3-(2,3,6-trifluorobenzyl)imidazo[1,5-a]pyridin-1-yl]]-5,5-dimethyl-5,7-dihydro-6H-pyrrolo[2,3-d]pyrimidin-6-on (23), 4-Amino-2-[6-fluor-3-(2,3,6-trifluorobenzyl)6-fluoroimidazo[1,5-a]pyridin-1-yl]-5,5-dimethyl-5,7-dihydro-6H-pyrrolo[2,3-d]pyrimidin-6-o n (24), 4-Amino-5,5-dimethyl-2-[3-(2,4,6-trifluorobenzyl)imidazo[1,5-a]pyridin-1-yl]]-5,7-dihydro-6H-pyrrolo[2,3-d]pyrimidin-6-on (25), 4-Amino-2-[3-(2-cyclopentylethyl)imidazo[1,5-a]pyridin-1-yl]-5,5-dimethyl-5,7-dihydro-6H-pyrrolo[2,3-d]pyrimidin-6-on (26), offenbart in WO 2010/065275.

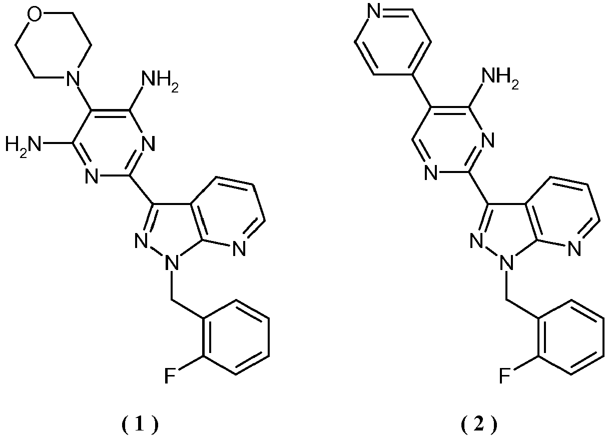

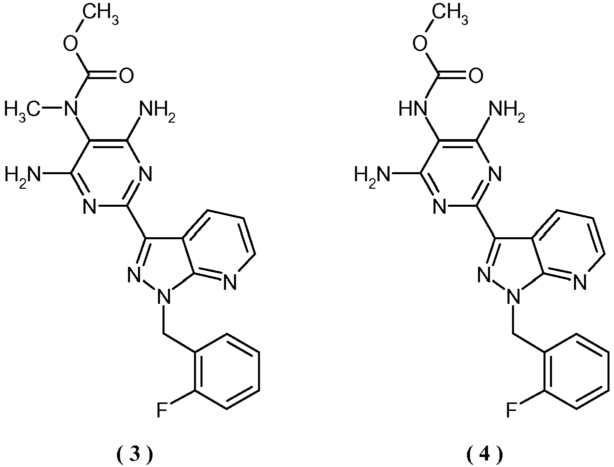

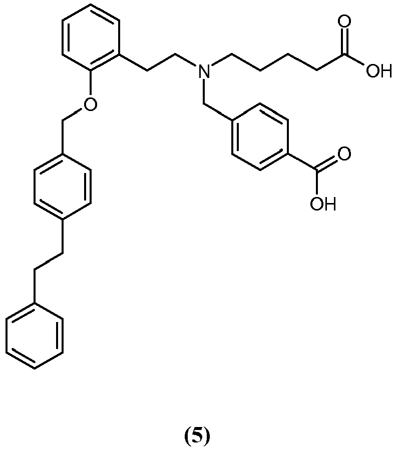

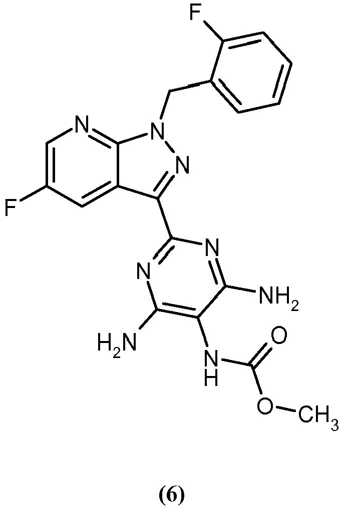

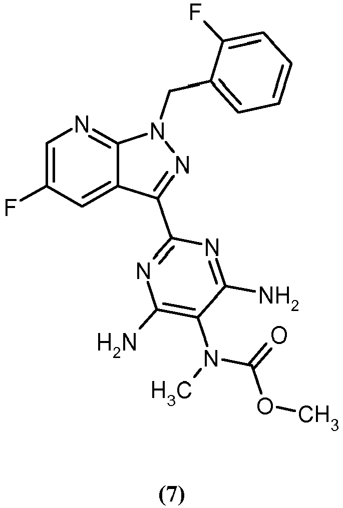

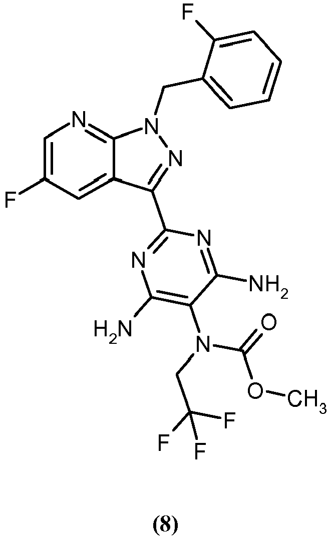

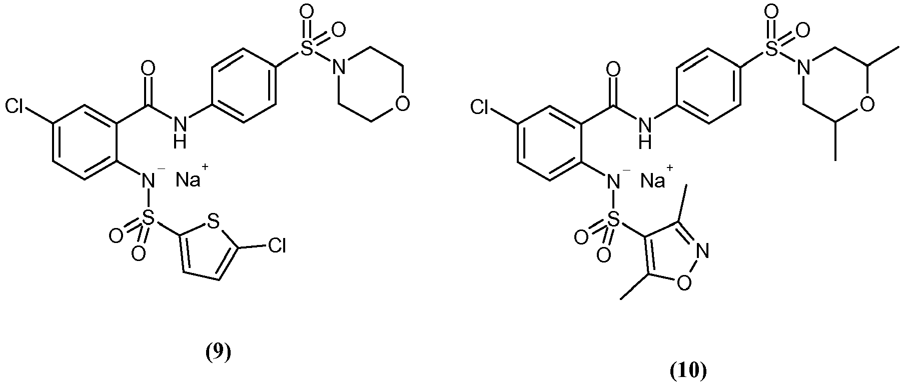

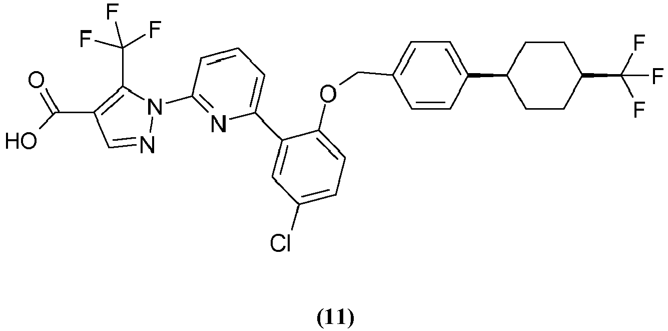

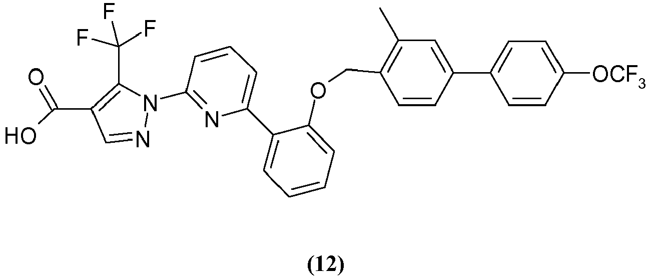

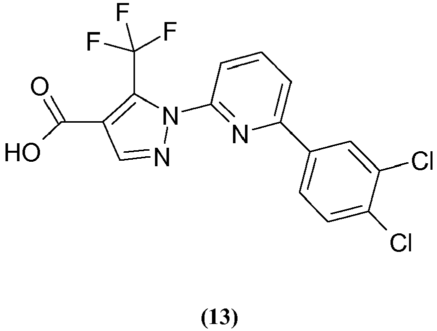

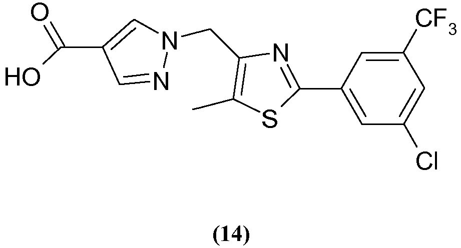

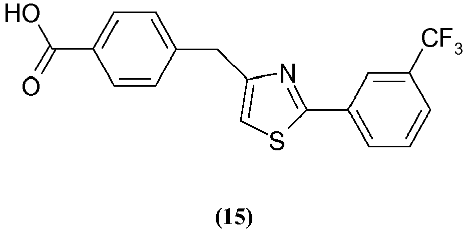

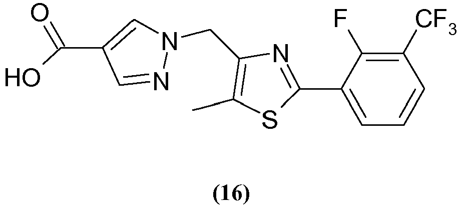

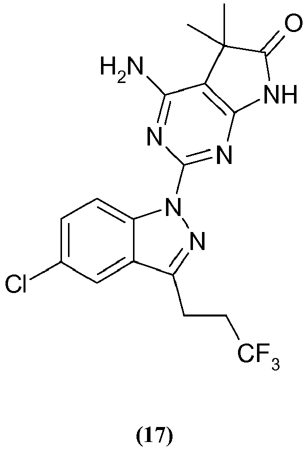

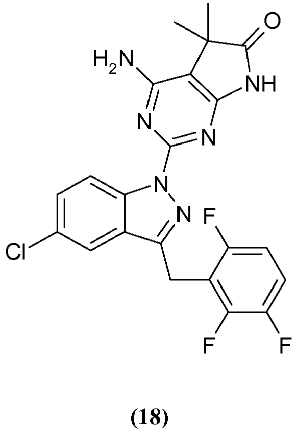

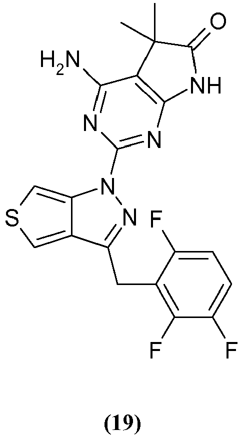

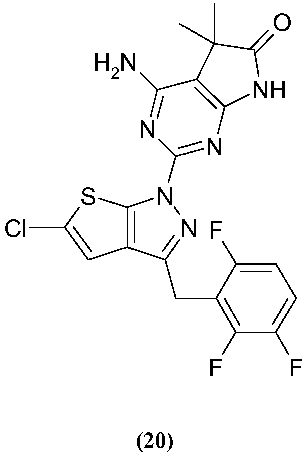

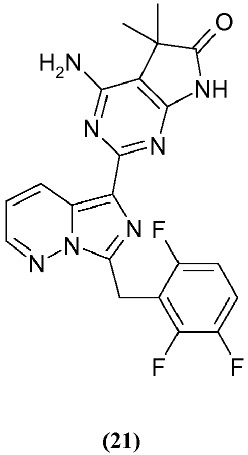

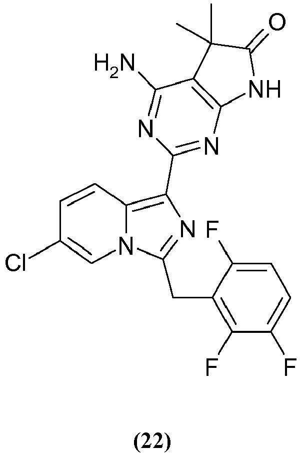

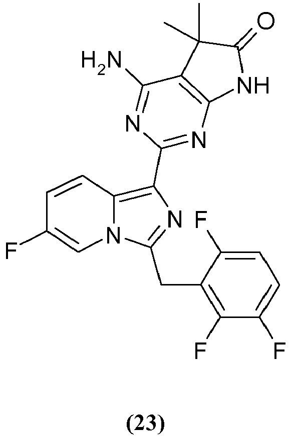

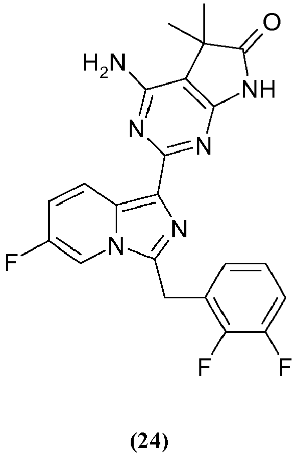

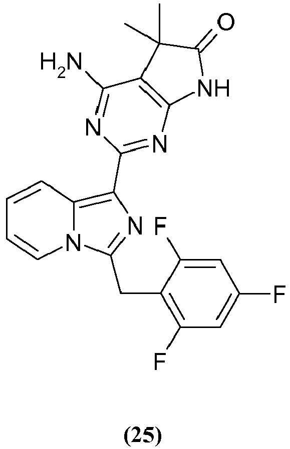

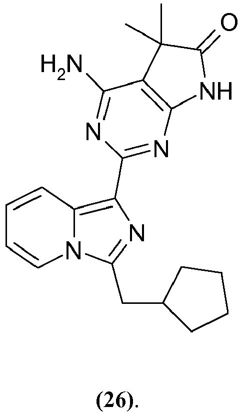

Verbindungen der Formeln (1), (2), (3), (4), (6)-(8) und (17)-(26) sind bekannt als Stimulatoren der löslichen Guanylatzyklase. Besonders bevorzugt sind die Verbindungen der Formeln (3), (4), (6) und (7).

Verbindungen der Formeln (5) und (9)-(16) sind bekannt als Aktivatoren der löslichen Guanylatzyklase. Besonders bevorzugt ist die Verbindung der Formel (5).

Ein weiterer Gegestand der Erfindung ist die Kombination von Stimulatoren und/oder Aktivatoren der löslichen Guanylatcyclase mit PDE5 Inhibitoren zur Verwendung zur Behandlung von Sichelzellanämie und Konservierung von Blutersatzstoffen.

Die im folgenden aufgelisteten PDE5 Inhibitoren eignen sich für die Kombination zur Verwendung zur Behandlung von Sichelzellanämie und Konservierung von Blutersatzstoffen:

*Tadalafil* ((6R,12aR) -2,3,6,7,12,12a - Hexahydro - 2 - methyl - 6 - (3,4-methylene -dioxyphenyl) pyrazino(1',2':1,6) pyrido(3,4-b)indole-1,4-dione), *Vardenafil* (2-(2-Ethoxy-5-(4-ethylpiperazin-1-yl-1-sulfonyl)phenyl)-5-methyl-7-propyl-3H-imidazo (5,1-f) (1,2,4)triazin-4-one), *Sildenafil* (3-[2-ethoxy-5-(4-methylpiperazin-1-yl)sulfonyl-phenyl]- 7- methy 1- 9- propy 1-2,4,7,8- tetrazabicyclo [4.3.0]nona -3,8,10-trien-5-one), *Udenafil* 5-[2-propyloxy-5-(1-methyl-2-pyrrolidinylethylamidosulfonyl)phenyl]-methyl-3-propyl-1,6-dihydro-7H-pyrazolo(4,3-d)pyrimidine-7-one, *Dasantafil* 7-(3-Bromo-4-methoxybenzyl)-1-ethyl-8-[[(1,2)-2-hydroxycyclopentyl]amino]-3-(2-hydroxyethyl)-3,7-dihydro-1-purine-2,6-dione, *Avanafil* 4-{[(3-chloro-4-methoxyphenyl)methyl]amino}-2-[(2S)-2-(hydroxymethyl)pyrrolidin-1-yl]-N-(pyrimidin-2-ylmethyl)pyrimidine-5-carboxamide, Mirodenafil, Lodenafil, UK 369.003, UK 371.800, *SLx 2101* of Surface Logix, *LAS 34179*Triazolo[1,2-]xanthine,6-methyl-4-propyl-2-[2-propoxy-5-(4-methylpiperazino)sulfonyl]phenyl oder Salze, Hydrate oder Hydrate der Salze davon.

Besonders bevorzugt sind Kobinationen von Verbindungen der Formeln (3), (4), (6), (7) und /oder (5) mit Vardenafil und/oder Sildenafil.

Gegenstand der vorliegenden Erfindung ist ein Verfahren zur Behandlung und/oder Prophylaxe von Sichelzellanämie und Konservierung von Blutersatzstoffen unter Verwendung einer therapeutisch wirksamen Menge einer oder mehrerer der erfindungsgemäßen Verbindung gemäß der Formeln (1) bis (26).

Ein weiterer Gegenstand der vorliegenden Erfindung ist die Verwendung von einer oder mehrerer der erfindungsgemäßen Verbindungen gemäß der Formeln (1) bis (26) zur Herstellung eines Arzneimittels zur Behandlung und/oder Prophylaxe von Sichelzellanämie und Konservierung von Blutersatzstoffen.

Ein weiterer Gegenstand der vorliegenden Erfindung ist ein Arzneimittel, enthaltend eine oder mehrere erfindungsgemäße Verbindung der Formeln (1) bis (26) und einen oder mehrere weitere Wirkstoffe, insbesondere zur Behandlung und/oder Prophylaxe von Sichelzellanämie und Konservierung von Blutersatzstoffen.

Ein weiterer Gegenstand der vorliegenden Erfindung ist die Verwendung von einer oder mehrerer Verbindungen der der Formeln (1) bis (26) in Kombination mit einem oder mehreren der folgenden PDE5 Inhibitoren: *Tadalafil* ((6R,12aR) -2,3,6,7,12,12a - Hexahydro - 2 - methyl - 6 - (3,4-methylene -dioxyphenyl) pyrazino(1',2':1,6) pyrido(3,4-b)indole-1,4-dione), *Vardenafil* (2-(2-Ethoxy-5-(4-ethylpiperazin-1-yl-1-sulfonyl)phenyl)-5-methyl-7-propyl-3H-imidazo (5,1-f) (1,2,4)triazin-4-one), *Sildenafil* (3-[2-ethoxy-5-(4-methylpiperazin-1-yl)sulfonyl-phenyl]- 7- methy 1-9- propy 1-2,4,7,8- tetrazabicyclo [4.3.0]nona -3,8,10-trien-5-one), *Udenafil* 5-[2-propyloxy-5-(1-methyl-2-pyrrolidinylethylamidosulfonyl)phenyl]-methyl-3-propyl-1,6-dihydro-7H-pyrazolo(4,3-d)pyrimidine-7-one, *Dasantafil* 7-(3-Bromo-4-methoxybenzyl)-1-ethyl-8-[[(1,2)-2-hydroxycyclopentyl]amino]-3-(2-hydroxyethyl)-3,7-dihydro-1-purine-2,6-dione, *Avanafil* 4-{[(3-chloro-4-methoxyphenyl)methyl]amino}-2-[(2S)-2-(hydroxymethyl)pyrrolidin-1-yl]-N-(pyrimidin-2-ylmethyl)pyrimidine-5-carboxamide, Mirodenafil, Lodenafil, UK 369.003, UK 371.800, *SLx 2101* of Surface Logix, *LAS 34179*Triazolo[1,2-]xanthine,6-methyl-4-propyl-2-[2-propoxy-5-(4-methylpiperazino)sulfonyl]phenyl oder Salze, Hydrate oder Hydrate der Salze davon zur Behandlung und/oder Prophylaxe von Sichelzellanämie und Konservierung von Blutersatzstoffen.

Ein weiterer Gegenstand der vorliegenden Erfindung ist die Verwendung von einer oder mehrerer Verbidnungen der Formeln (3) bis (7) gemäß Anspruch 1 in Kombination mit Vardenafil und/oder Sildenafil zur Behandlung und/oder Prophylaxe von Sichelzellanämie und Konservierung von Blutersatzstoffen.

Ein weiterer Gegenstand der vorliegenden Erfindung ist die Verwendung von einer oder mehrerer der Verbindungen der Formeln (1) bis (26) in Kombination mit den oben genannten PDE5 Inhibitoren zur Behandlung und/oder Prophylaxe von traumatisierten Patienten, welche einen künstlichen Blutersatzstoff erhalten.

Ein weiterer Gegenstand der vorliegenden Erfindung ist ein Verfahren zur Behandlung und/oder Prophylaxe von Sichelzellanämie und Konservierung von Blutersatzstoffen unter Verwendung einer therapeutisch wirksamen Menge einer oder mehrerer erfindungsgemäßen Verbindung gemäß der Formeln (1) bis (26) in Kombination mit den oben genannten PDE5 Inhibitoren.

Ein weiterer Gegenstand der vorliegenden Erfindung ist die Verwendung einer oder mehrerer der erfindungsgemäßen Verbindungen gemäß der Formeln (1) bis (26) in Kombination mit den oben genannten PDE5 Inhibitoren zur Herstellung eines Arzneimittels zur Behandlung und/oder Prophylaxe von Sichelzellanämie und Konservierung von Blutersatzstoffen.

Ein weiterer Gegenstand der vorliegenden Erfindung ist ein Arzneimittel, enthaltend eine oder mehrere der erfindungsgemäßen Verbindungen der Formeln (1) bis (26) in Kombination mit dem oben genannten PDE5 Inhibitoren und einen oder mehrere weitere Wirkstoffe, insbesondere zur Behandlung und/oder Prophylaxe von Sichelzellanämie und Konservierung von Blutersatzstoffen.

Ein weiterer Gegenstand der vorliegenden Erfindung ist die Verwendung von einer oder mehrerer Verbindungen der Formeln (1) bis (26) zur Behandlung und/oder Prophylaxe von Sichelzellanämie wobei die Anwendung inhalativ erfolgt.

Ein weiterer Gegenstand der vorliegenden Erfindung Verwendung von einer oder mehrerer Verbindungen der Formeln (1) bis (26) in Kombination mit den oben genannten PDE5 Inhibitoren zur Behandlung und/oder Prophylaxe von Sichelzellanämie wobei die Anwendung inhalativ erfolgt.

Darüber hinaus wurden NO- und Häm-unabhängige sGC-Aktivatoren, mit der Verbindung der Formel (5) als Prototyp dieser Klasse, identifiziert. Gemeinsame Charakteristika dieser Substanzen sind, dass sie in Kombination mit NO nur einen additiven Effekt auf die Enzymaktivierung ausüben, und dass die Aktivierung des oxidierten oder hämfreien Enzyms im Vergleich zum hämhaltigen Enzym deutlich stärker ist [Evgenov et al., *ibid*.; J.P. Stasch et al., Br. J. Pharmacol. 136 (2002), 773; J.P. Stasch et al., J. Clin. Invest. 116 (2006), 2552]. Spektroskopische Untersuchungen lassen erkennen, dass die Verbindung der Formel (5) die oxidierte Hämgruppe verdrängt, die durch Schwächung der Eisen-Histidin-Bindung nur schwach an der sGC gebunden ist. Auch wurde gezeigt, dass das charakteristische sGC-Hämbindungsmotiv Tyr-x-Ser-x-Arg sowohl für die Interaktion der negativ geladenen Propionsäuren der Hämgruppe als auch für die Wirkung von die Verbindung der Formel (5) zwingend erforderlich ist. Vor diesem Hintergrund wird angenommen, dass die Bindungsstelle von die Verbindung der Formel (5) an der sGC identisch zur Bindungsstelle der Hämgruppe ist [J.P. Stasch et al., J. Clin. Invest. 116 (2006), 2552]. Die in der vorliegenden Erfindung beschriebenen Verbindungen sind nun ebenfalls in der Lage, die Häm-freie Form der löslichen Guanylatcyclase zu aktivieren. Dies wird auch dadurch belegt, dass diese neuartigen Aktivatoren einerseits am Häm-haltigen Enzym keine synergistische Wirkung mit NO zeigen und andererseits sich ihre Wirkung nicht durch den Häm-abhängigen Inhibitor der löslichen Guanylatcyclase, 1*H*-1,2,4-Oxadiazolo[4,3-a]chinoxalin-1-on (ODQ), blockieren lässt, sondern durch diesen sogar potenziert wird [vgl. O.V. Evgenov et al., Nature Rev. Drug Disc. 5 (2006), 755; J.P. Stasch et al., J. Clin. Invest. 116 (2006), 2552] und hier als sGC Aktivatoren im Sinne dieser Erfindung zu verstehen sind. Dazu sollen insbesondere folgende sGC Aktivatorklassen zählen gemäß den Verbindungen der Formeln (5) und (9)-(16).

Die erfindungsgemäßen Verbindungen zeigen ein nicht vorhersehbares, wertvolles pharmakologisches und pharmakokinetisches Wirkspektrum. Neben der Verwendung zur Behandlung von Sichelzellanämie und Konservierung von Blutersatzstoffen führen die sGC Stimulatoren und/oder Aktivatoren zu einer Gefäßrelaxation und/oder zu einer Thrombozytenaggregationshemmung und/oder zu einer Blutdrucksenkung und/oder zu einer Steigerung des koronaren Blutflusses sowie der Mikrozirkulation generell führen. Sie eigenen sich daher zur Behandlung und/oder Prophylaxe von Krankheiten, vorzugsweise von kardiovaskulären Erkrankungen, bei Menschen und Tieren.

Die erfindungsgemäßen Verbindungen eignen sich daher auch zur Behandlung von kardiovaskulären Erkrankungen wie beispielsweise zur Behandlung des Bluthochdrucks, der primären und/oder sekundären Prävention sowie Behandlung der Herzinsuffizienz, zur Behandlung stabiler und instabiler Angina pectoris, pulmonaler Hypertonie, peripheren und kardialen Gefäßerkrankungen, Arrhythmien, zur Behandlung von thromboembolischen Erkrankungen und Ischämien wie Myokardinfarkt, Hirnschlag, transistorischen und ischämischen Attacken, peripheren Durchblutungsstörungen, zur Verhinderung von Restenosen wie nach Thrombolysetherapien, percutantransluminalen Angioplastien (PTA), percutan-transluminalen Koronarangioplastien (PTCA) und Bypass sowie zur Behandlung von Arteriosklerose, asthmatischen Erkrankungen, Krankheiten des Urogenitalsystems wie beispielsweise Prostatahypertrophie, erektile Dysfunktion, weibliche sexuelle Dysfunktion und Inkontinenz eingesetzt werden.

Außerdem können die erfindungsgemäßen Verbindungen zur Behandlung von primärem und sekundärem Raynaud-Phänomen, von Mikrozirkulationsstörungen, Claudicatio, peripheren und autonomen Neuropathien, diabetischen Mikroangiopathien, diabetischer Nephropathie, diabetischer Retinopathie, diabetischen Geschwüren an den Extremitäten, CREST-Syndrom, Erythematose, Onychomykose, Tinnitus, Schwindelanfälle, Hörsturz sowie von rheumatischen Erkrankungen verwendet werden.

Ferner eignen sich die erfindungsgemäßen Verbindungen zur Behandlung von Respiratory Distress-Syndromen und chronisch-obstruktiven Atemwegserkrankungen (COPD), von akuter und chronischer Niereninsuffizienz sowie zur Förderung der Wundheilung.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen eingesetzt werden.

Darüber hinaus können die erfindungsgemäßen Verbindungen auch zur Behandlung und/oder Prävention von mikro- und makrovaskulären Schädigungen (Vasculitis), Reperfusionsschäden, arteriellen sowie venösen Thrombosen, Ödemen, Krebserkrankungen (Hautkrebs, Liposarcome, Karzinome des Magen-Darm-Traktes, der Leber, Bauchspeicheldrüse, Lunge, Niere, Harnleiter, Prostata und des Genitaltraktes), von Erkrankungen des Zentralen Nervensystems und neurodegenerativen Störungen (Schlaganfall, Alzheimer'sche Krankheit, Parkinson'sche Krankheit, Demenz, Epilepsie, Depressionen, Multiple Sklerose), von Entzündungserkrankungen, Immunerkrankungen (Morbus Crohn, Colitis ulcerosa, Lupus erythematodes, rheumatoide Arthritis, Asthma), Nierenerkrankungen (Glomerulonephritis), Schilddrüsenerkrankungen (Hyperthyreose), Erkrankungen der Bauchspeicheldrüse (Pankreatitis), Leberfibrose, Hauterkrankungen (Psoriasis, Akne, Ekzeme, Neurodermitis, Dermatitis, Keratitis, Narbenbildung, Warzenbildung, Frostbeulen), viralen Erkrankungen (HPV, HCMV, HIV), Kachexie, Osteoporose, Gicht, Inkontinenz sowie zur Wundheilung und Angiogenese eingesetzt werden.

Weiterer Gegenstand der vorliegenden Erfindung ist der Einsatz der erfindungsgemäßen Verbindungen zur Behandlung und/oder Prophylaxe von Erkrankungen, vorzugsweise von thromboembolischen Erkrankungen und/oder thromboembolischen Komplikationen.

Zu den "thromboembolischen Erkrankungen" im Sinne der vorliegenden Erfindung zählen insbesondere Erkrankungen wie Herzinfarkt mit ST-Segment-Erhöhung (STEMI) und ohne ST-Segment-Erhöhung (non-STEMI), stabile Angina Pectoris, instabile Angina Pectoris, Reokklusionen und Restenosen nach Koronarinterventionen wie Angioplastie, Stentimplantation oder aortokoronarem Bypass, periphere arterielle Verschlusskrankheiten, Lungenembolien, tiefe venöse Thrombosen und Nierenvenenthrombosen, transitorische ischämische Attacken sowie thrombotischer und thromboembolischer Hirnschlag und pulmonale Hypertonie.

Die Substanzen eignen sich daher auch zur Prävention und Behandlung von kardiogenen Thromboembolien, wie beispielsweise Hirn-Ischämien, Schlaganfall (Stroke) und systemischen Thromboembolien und Ischämien, bei Patienten mit akuten, intermittierenden oder persistierenden Herzarrhythmien, wie beispielsweise Vorhofflimmern, und solchen, die sich einer Kardioversion unterziehen, ferner bei Patienten mit Herzklappen-Erkrankungen oder mit intravasalen Körpern, wie z. B. künstlichen Herzklappen, Kathetern, intraaortale Ballongegenpulsation und Schrittmachersonden. Darüber hinaus sind die erfindungsgemäßen Verbindungen zur Behandlung der disseminierten intravasalen Gerinnung (DIC) geeignet.

Thromboembolische Komplikationen treten ferner auf bei mikroangiopathischen hämolytischen Anämien, Sichelzellanämie, Thalassämie, vererbten Formen von Spherozytose, Eliptozytose und Ovalzytose, Malaria, Moskowitch Syndrom, hämolytisches Uraemie Syndrom, extrakorporalen Blutkreisläufen, wie z. B. Hämodialyse, Hämofiltration, ventricular assist devices und Kunstherz, sowie Herzklappenprothesen, Bluttransfusionen, beim Kardiopulmonalen Bypass und Organtransplantationen (z.B. Lunge, Herz, Niere, Leber), Hämolyse auf Grund von intravasculären Devices oder durch Hämodialyse sowie bei der Anwendung von künstlichen Hb-basierten Sauerstoffträgern.

Besonders eignen sich die erfindungsgemäßen Verbindungen auch zur primären und/oder sekundären Prävention sowie Behandlung der Herzinsuffizienz.

Im Sinne der vorliegenden Erfindung umfasst der Begriff Herzinsuffizienz auch spezifischere oder verwandte Krankheitsformen wie Rechtsherzinsuffizienz, Linksherzinsuffizienz, Globalinsuffizienz, ischämische Kardiomyopathie, dilatative Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz. Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Desweiteren bietet sich die vorliegende Erfindung auch für die Behandlung von traumatisierten Patienten, welche einen künstlichen Blutersatzstoff erhalten [Weisskopf 2010] an.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer therapeutisch wirksamen Menge einer erfindungsgemäßen Verbindung.

Weiterer Gegenstand der vorliegenden Erfindung ist die Verwendung der erfindungsgemäßen Verbindungen zur Herstellung eines Arzneimittels zur Behandlung und/oder Prophylaxe von Sichelzellanämie und Konservierung von Blutersatzstoffen.

Weiterer Gegenstand der vorliegenden Erfindung ist ein Verfahren zur Behandlung und/oder Prophylaxe von Sichelzellanämie und Konservierung von Blutersatzstoffen, unter Verwendung einer therapeutisch wirksamen Menge einer erfindungsgemäßen Verbindung.

Die erfindungsgemäßen Verbindungen können allein oder bei Bedarf in Kombination mit anderen Wirkstoffen eingesetzt werden.

Weiterer Gegenstand der vorliegenden Erfindung sind Arzneimittel, enthaltend eine erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt: den Fettstoffwechsel verändernde Wirkstoffe, Antidiabetika, Blutdruck-Senker, durchblutungsfördernd und/oder antithrombotisch wirkende Mittel sowie Antioxidantien, Aldosteron- und Mineralokortikoid-Rezeptor-Antagonisten, Vasopressin-Rezeptor-Antagonisten, organische Nitrate und NO-Donatoren, IP- Rezeptor Agonisten, positiv inotrop wirksamen Verbindungen, ACE Inhibitoren, cGMP und cAMP modulierende Verbindungen, Inhibitoren der neutrophilen Elastase, die Signaltransduktionskaskade inhibierende Verbindungen, den Energiestoffwechsel des Herzens modulierende Verbindungen, Chemokin-Rezeptor-Antagonisten, p38-Kinase-Inhibitoren, NPY-Agonisten, Orexin-Agonisten, Anorektika, PAF-AH-Inhibitoren, Antiphlogistika (COX-Inhibitoren, LTB₄-Rezeptor-Antagonisten, LTB₄-Synthese Hemmer), Analgetika (Aspirin), Antidepressiva und andere Psychopharmaka.

Gegenstand der vorliegenden Erfindung sind insbesondere Kombinationen mit mindestens einer der erfindungsgemäßen Verbindungen mit mindestens einem den Fettstoffwechsel verändernden Wirkstoff, einem Antidiabetikum, einem blutdrucksenkenden Wirkstoff und/oder einem antithrombotisch wirkenden Mittel.

Die erfindungsgemäßen Verbindungen können vorzugsweise mit einem oder mehreren
- den Fettstoffwechsel verändernden Wirkstoffen, beispielhaft und vorzugsweise aus der Gruppe der HMG-CoA-Reduktase-Inhibitoren aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, Inhibitoren der HMG-CoA-Reduktase-Expression, Squalensynthese-Inhibitoren wie beispielhaft und vorzugsweise BMS-188494 oder TAK-475, ACAT-Inhibitoren wie beispielhaft und vorzugsweise Melinamide, Pactimibe, Eflucimibe oder SMP-797, LDL-Rezeptor-Induktoren, Cholesterin-Absorptionshemmer wie beispielhaft und vorzugsweise Ezetimibe, Tiqueside oder Pamaqueside, polymeren Gallensäureadsorber, wie beispielhaft und vorzugsweise Cholestyramin, Colestipol, Colesolvam, CholestaGel oder Colestimide, Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635" MTP-Inhibitoren wie beispielhaft und vorzugsweise Implitapide oder JTT-130, Lipase-Inhibitoren wie beispielhaft und vorzugsweise Orlistat, LpL-Aktivatoren, Fibrate, Niacin, CETP-Inhibitoren, wie beispielhaft und vorzugsweise Torcetrapib, JTT-705 oder CETP vaccine (Avant), PPAR-γ- und/oder PPAR-δ-Agonisten, wie beispielhaft und vorzugsweise Pioglitazone oder Rosiglitazone und/oder GW-501516, RXR-Modulatoren, FXR-Modulatoren, LXR-Modulatoren, Thyroidhormone und/oder Thyroidmimetika wie beispielhaft und vorzugsweise D-Thyroxin oder 3,5,3'-Triiodothyronin (T3), ATP-Citrat-Lyase-Inhibitoren, Lp(a)-Antagonisten, Cannabinoid-Rezeptor 1-Antagonisten, wie beispielhaft und vorzugsweise Rimonabant oder SR-147778, Leptin-Rezeptor-Agonisten, Bombesin-Rezeptor-Agonisten, Histamin-Rezeptor-Agonisten, Agonisten des Niacin-Rezeptors, wie beispielhaft und vorzugsweise Niacin, Acipimox, Acifran oder Radecol, sowie der Antioxidantien / Radikalfänger wie beispielhaft und vorzugsweise Probucol, AGI-1067, BO-653 oder AEOL-10150,
- Antidiabetika, die in der Roten Liste 2009/I, Kapitel 12 genannt sind. Unter Antidiabetika werden vorzugsweise Insulin und Insulinderivate sowie oral wirksame hypoglykämische Wirkstoffe verstanden. Insulin und Insulinderivate umfasst hierbei sowohl Insuline tierischen, menschlichen oder biotechnologischen Ursprungs als auch Gemische hieraus. Die oral wirksamen hypoglykämischen Wirkstoffe umfassen vorzugsweise Sulphonylharnstoffe, Biguanide, Meglitinid-Derivate, Glukosidase-Inhibitoren und PPAR-gamma-Agonisten sowie beispielhaft und vorzugsweise jenen aus der Gruppe der Sulphonylharnstoffe, wie beispielhaft und vorzugsweise Tolbutamid, Glibenclamid, Glimepirid, Glipizid oder Gliclazid, Biguanide, wie beispielhaft und vorzugsweise Metformin, Meglitinid-Derivate, wie beispielhaft und vorzugsweise Repaglinid oder Nateglinid, Glukosidase-Inhibitoren, wie beispielhaft und vorzugsweise Miglitol oder Acarbose, Oxadiazolidinone, Thiazolidindione, GLP 1-Rezeptor-Agonisten, Glukagon-Antagonisten, Insulin-Sensitizer, CCK 1-Rezeptor-Agonisten, Leptin-Rezeptor-Agonisten, Inhibitoren von Leberenzymen, die an der Stimulation der Glukoneogenese und/oder Glykogenolyse beteiligt sind, Modulatoren der Glukoseaufnahme sowie der Kaliumkanalöffner, wie z.B. denjenigen, die in WO 97/26265 und WO 99/03861 offenbart sind;
- den Blutdruck senkenden Wirkstoffen, beispielhaft und vorzugsweise aus der Gruppe der Calcium-Antagonisten wie beispielhaft und vorzugsweise Nifedipin, Amlodipin, Verapamil oder Diltiazem, Angiotensin All-Antagonisten, wie beispielhaft und vorzugsweise Losartan, Valsartan, Candesartan, Embusartan oder Telmisartan, ACE-Inhibitoren, wie beispielhaft und vorzugsweise Enalapril, Captopril, Ramipril, Delapril, Fosinopril, Quinopril, Perindopril oder Trandopril, beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, alpha-Rezeptoren-Blocker, wie beispielhaft und vorzugsweise Prazosin, ECE-Inhibitoren, Rhokinase Inhibitoren und der Vasopeptidase-Inhibitoren, sowie der Diuretika, wie beispielhaft und vorzugsweise einem Schleifendiuretikum wie Furosemid, Bumetanid oder Torsemid, oder einem Thiazid- oder Thiazid-ähnlichen Diuretikum wie Chlorthiazid oder Hydrochlorthiazid oder A1 Antagonisten wie Rolofylline, Tonopofylline und SLV-320;
- den Sympathikustonus senkende Mittel wie beispielhaft und vorzugsweise Reserpin, Clonidin oder alpha-Methyl-Dopa, oder in Kombination mit einem Kaliumkanal-Agonisten, wie beispielhaft und vorzugsweise Minoxidil, Diazoxid, Dihydralazin oder Hydralazin,
- antithrombotisch wirkenden Mitteln, beispielhaft und vorzugsweise aus der Gruppe der Thrombozytenaggregationshemmer, wie beispielhaft und vorzugsweise Aspirin, Clopidogrel, Ticlopidin, Cilostazol oder Dipyridamol, oder der Antikoagulantien wie Thrombin-Inhibitoren, wie beispielhaft und vorzugsweise Ximelagatran, Melagatran, Bivalirudin oder Clexane, einem GPIIb/IIIa-Antagonisten, wie beispielhaft und vorzugsweise Tirofiban oder Abciximab, einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, mit Heparin oder einem low molecular weight (LMW)-Heparin-Derivat oder mit einem Vitamin K-Antagonisten, wie beispielhaft und vorzugsweise Coumarin,

- Aldosteron- und Mineralokorticoid-Rezeptor-Antagonisten, wie beispielhaft und vorzugsweise Spironolacton oder Eplerenon;
- Vasopressin-Rezeptor-Antagonisten, wie beispielhaft und vorzugsweise Conivaptan, Tolvaptan, Lixivaptan oder SR-121463,;
- organischen Nitraten und NO-Donatoren wie beispielhaft und vorzugweise Natriumnitroprussid, Nitroglycerin, Isosorbidmononitrat, Isosorbiddinitrat, Molsidomin oder SIN-1, oder in Kombination mit inhalativem NO;
- IP-Rezeptor Agonisten wie wie beispielhaft und vorzugweise Iloprost, Treprostinil, Beraprost und NS-304
- positiv-inotrop wirksamen Verbindungen, wie beispielhaft und vorzugsweise Herzglycosiden (Digoxin), beta-adrenergen und dopaminergen Agonisten wie Isoproterenol, Adrenalin, Noradrenalin, Dopamin oder Dobutamin,;
- Calcium-Sensitizern, wie beispielhaft und vorzugsweise Levosimendan;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil sowie PDE 3-Inhibitoren wie Milrinone;
- natriuretischen Peptiden, wie z.B. "atrial natriuretic peptide" (ANP, Anaritide), "B-type natriuretic peptide" oder "brain natriuretic peptide" (BNP, Nesiritide), "C-type natriuretic peptide" (CNP) sowie Urodilatin;
- NO-unabhängigen, jedoch Häm-abhängigen Stimulatoren der Guanylatcyclase, wie insbesondere den in WO 00/06568, WO 00/06569, WO 02/42301 und WO 03/095451 beschriebenen Verbindungen;
- NO- und Häm-unabhängigen Aktivatoren der Guanylatcyclase, wie insbesondere den in WO 01/19355, WO 01/19776, WO 01/19778, WO 01/19780, WO 02/070462 und WO 02/070510 beschriebenen Verbindungen;
- Inhibitoren der humanen neutrophilen Elastase (HNE), wie beispielsweise Sivelestat und DX-890 (Reltran);
- die Signaltransduktionskaskade inhibierenden Verbindungen, wie beispielsweise Tyrosinkinase-Inhibitoren und Multikinase Inhibitoren, insbesondere Sorafenib, Imatinib, Gefitinib und Erlotinib; und/oder
- den Energiestoffwechsel des Herzens beeinflussenden Verbindungen, wie beispielweise Etomoxir, Dichloracetat, Ranolazine und Trimetazidine
kombiniert werden.

Besonders bevorzugt im Rahmen der vorliegenden Erfindung sind Kombinationen enthaltend mindestens eine der erfindungsgemäßen Verbindungen sowie einen oder mehrere weitere Wirkstoffe ausgewählt aus der Gruppe bestehend aus HMG-CoA-Reduktase-Inhibitoren (Statine), Diuretika, Antidiabetika, beta-Rezeptoren-Blocker, organische Nitrate und NO-Donatoren, ACE-Inhibitoren, Angiotensin AII-Antagonisten, Aldosteron- und Mineralokortikoid-Rezeptor-Antagonisten, Vasopressin-Rezeptor-Antagonisten, Thrombozytenaggregationshemmer und Antikoagulantien, sowie deren Verwendung zur Behandlung und/oder Prävention der zuvor genannten Erkrankungen.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nicht überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Bevorzugt ist die orale Applikation.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Ebenfalls bevorzugt ist die inhalative Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, vorzugsweise zusammen mit einem oder mehreren inerten nichttoxischen, pharmazeutisch geeigneten Hilfsstoff enthalten, sowie deren Verwendung zur Behandlung von Sichelzellanämie und Konservierung von Blutersatzstoffen.

Im Allgemeinen hat es sich als vorteilhaft erwiesen, bei parenteraler Applikation Mengen von etwa 5 bis 100 mg je 24 Stunden zur Erzielung wirksamer Ergebnisse zu verabreichen. Bei oraler Applikation beträgt die Menge etwa 5 bis 250 mg je 24 Stunden.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen. Die Angabe "w/v" bedeutet "weight/volume" (Gewicht/Volumen). So bedeutet beispielsweise "10% w/v": 100 ml Lösung oder Suspension enthalten 10 g Substanz.

### Experimenteller Teil

### A. Beispiele

### Abkürzungen und Akronyme:

- aq.: wässrige Lösung
- ber.: Berechnet
- DCI: direkte chemische Ionisation (bei MS)
- DMF: Dimethylformamid
- DMSO: Dimethylsulfoxid
- d. Th.: der Theorie (bei Ausbeute)
- eq.: Äquivalent(e)
- ESI: Elektrospray-Ionisation (bei MS)
- Et: Ethyl
- gef.: Gefunden
- h: Stunde(n)
- HPLC: Hochdruck-, Hochleistungsflüssigchromatographie
- HRMS: hochaufgelöste Massenspektrometrie
- konz.: konzentriert
- LC/MS: Flüssigchromatographie-gekoppelte Massenspektrometrie
- LiHMD S: Lithiumhexamethyldisilazid
- Me: Methyl
- min: Minute(n)
- MS: Massenspektrometrie
- NMR: Kernresonanzspektrometrie
- Pd₂dba₃: Tris-(dibenzylidenaceton)-dipalladium
- Ph: Phenyl
- RT: Raumtemperatur
- Rₜ: Retentionszeit (bei HPLC)
- THF: Tetrahydrofuran
- UV: Ultraviolett-Spektrometrie
- v/v: Volumen zu Volumen-Verhältnis (einer Lösung)
- XPHOS: Dicyclohexyl-(2',4',6'-triisopropylbiphenyl-2-yl)-phosphin

### LC/MS-Methoden:

Methode 1: Gerätetyp MS: Waters ZQ; Gerätetyp HPLC: Agilent 1100 Series; UV DAD; Säule: Thermo Hypersil GOLD 3 µ 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 3.0 min 10% A → 4.0 min 10% A → 4.1 min 100% A (Fluss 2.5 ml/min); Ofen: 55°C; Fluss: 2 ml/min; UV-Detektion: 210 nm.
Methode 2: Instrument: Waters ACQUITY SQD UPLC System; Säule: Waters Acquity UPLC HSS T3 1.8 µ 50 x 1 mm; Eluent A: 1 1 Wasser + 0.25 ml 99%ige Ameisensäure , Eluent B: 1 1 Acetonitril + 0.25 ml 99%ige Ameisensäure; Gradient: 0.0 min 90% A → 1.2 min 5% A → 2.0 min 5% A Ofen: 50 °C; Fluss: 0.40 ml/min; UV-Detektion: 210 - 400 nm.

**Ausgangsverbindungen und Intermediate:**

### Beispiel 1A

### 2,6-Dichlor-5-fluornicotinamid

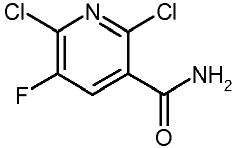

Eine Suspension aus 25 g (130.90 mmol) 2,6-Dichlor-5-fluor-3-cyanopyridin in konz. Schwefelsäure (125 ml) wurde 1 h bei 60-65°C gerührt. Nach Abkühlen auf RT wurde der Kolbeninhalt auf Eiswasser gegossen und dreimal mit Essigsäureethylester (je 100 ml) extrahiert. Die vereinigten organischen Phasen wurden mit Wasser (100 ml) und anschliessend mit gesättigter wässriger Natriumhydrogencarbonat-Lösung (100 ml) gewaschen, getrocknet und am Rotationsverdampfer eingeengt. Das erhaltene Material wurde am Hochvakuum getrocknet.

Ausbeute: 24.5 g (90 % d. Theorie)

¹H-NMR (400 MHz, DMSO-d₆): δ = 7.95 (br s, 1H), 8.11 (br s, 1H), 8.24 (d, 1H).

### Beispiel 2A

### 2-Chlor-5-fluornicotinamid

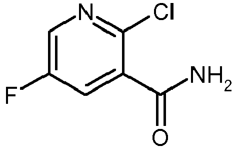

Einer Suspension von 21.9 g (335.35 mmol) Zink in Methanol (207 ml) wurden bei RT 44 g (210.58 mmol) 2,6-Dichlor-5-fluornicotinamid zugegeben. Danach wurde mit Essigsäure (18.5 ml) versetzt und unter Rühren für 24 h zum Rückfluss erhitzt. Danach wurde der Kolbeninhalt vom Zink dekantiert und Essigsäureethylester (414 ml) sowie gesättigte wässrige Natriumhydrogencarbonat-Lösung (414 ml) zugegeben und intensiv ausgerührt. Anschliessend wurde über Kieselgur abgesaugt und dreimal mit Essigsäureethylester (je 517 ml) nachgewaschen. Die organische Phase wurde abgetrennt und die wässrige Phase mit Essigsäureethylester (258 ml) gewaschen. Die vereinigten organischen Phasen wurden einmal mit gesättigter wässriger Natriumhydrogencarbonat-Lösung (414 ml) gewaschen, getrocknet und im Vakuum eingeengt. Die so erhaltenen Kristalle wurden mit Dichlormethan (388 ml) versetzt und 20 min. ausgerührt. Es wurde erneut abgesaugt und mit Diethylether nachgewaschen und trockengesaugt.

Ausbeute: 20.2 g (53 % d. Theorie)

¹H-NMR (400 MHz, DMSO-d₆): δ = 7.87 (br s, 1H), 7.99 (dd, 1H), 8.10 (br s, 1H), 8.52 (d, 1H).

### Beispiel 3A

### 2-Chlor-5-fluornicotinonitril

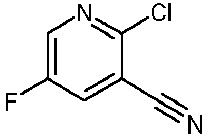

Eine Suspension von 46.2 g (264.66 mmol) 2-Chlor-5-fluornicotinamid in Dichlormethan (783 ml) wurde mit 81.2 ml (582.25 mmol) Triethylamin versetzt und auf 0°C gekühlt. Unter Rühren wurden dann 41.12 ml (291.13 mmol) Trifluoressigsäureanhydrid langsam zugetropft und 1.5 h bei 0°C nachgerührt. Die Reaktionslösung wurde danach zweimal mit gesättigter wässriger Natriumhydrogencarbonat-Lösung (je 391 ml) gewaschen, getrocknet und im Vakuum eingeengt.

Ausbeute: 42.1 g (90 % d. Theorie).

¹H-NMR (400 MHz, DMSO-d₆): δ = 8.66 (dd, 1H), 8.82 (d, 1H).

### Beispiel 4A

### 5-Fluor-1H-pyrazolo[3,4-b]pyridin-3-amin

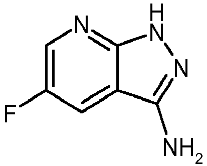

Eine Suspension aus 38.5 g (245.93 mmol) 2-Chlor-5-fluornicotinonitril wurde in 1,2-Ethandiol (380 ml) vorgelegt und danach mit Hydrazinhydrat (119.6 ml, 2.459 mol) versetzt. Es wurde unter Rühren für 4 h zum Rückfluss erhitzt. Beim Abkühlen fiel das Produkt aus. Die gelben Kristalle wurden mit Wasser (380 ml) versetzt und 10 min. bei RT ausgerührt. Anschliessend wurde die Suspension über eine Fritte abgesaugt, mit Wasser (200 ml) und mit -10°C-kaltem THF (200 ml) nachgewaschen. Der Rückstand wurde im Hochvakuum über Phosphorpentoxid getrocknet.

Ausbeute: 22.8 g (61 % d. Theorie)

¹H-NMR (400 MHz, DMSO-d₆): δ = 5.54 (s, 2H), 7.96 (dd, 1H), 8.38 (m, 1H), 12.07(m, 1H).

### Beispiel 5A

### 5-Fluor-3-iod-1H-pyrazolo[3,4-b]pyridin

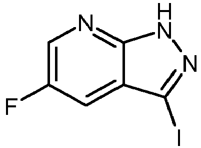

In THF (329 ml) wurden 10 g (65.75 mmol) 5-Fluor-1H-pyrazolo[3,4-b]pyridin-3-amin vorgelegt und auf 0°C abgekühlt. Anschliessend wurden 16.65 ml (131.46 mmol) Bortrifluorid-Diethylether-Komplex langsam zugesetzt. Die Reaktionsmischung wurde weiter auf -10°C abgekühlt. Danach wurde eine Lösung von 10.01 g (85.45 mmol) Isopentylnitrit in THF (24.39 ml) langsam zugefügt und weitere 30 min nachgerührt. Die Mischung wurde mit kaltem Diethylether (329 ml) verdünnt und der entstandene Feststoff abfiltriert. Das so hergestellte Diazoniumsalz wurde portionsweise in eine 0°C kalte Lösung von 12.81 g (85.45 mmol) Natriumiodid in Aceton (329 ml) gegeben und die Mischung 30 min bei RT nachgerührt. Die Reaktionsmischung wurde auf Eiswasser (1.81) gegeben und zweimal mit Essigsäureethylester (je 487 ml) extrahiert. Die gesammelten organischen Phasen wurden mit gesättigter wässriger Natriumchorid-Lösung (244 ml) gewaschen, getrocknet, filtriert und eingeengt. Man erhielt 12.1 g (86%-ige Reinheit, 60 % d. Th.) der gewünschten Verbindung als braunen Feststoff. Das Rohprodukt wurde ohne weitere Reinigung umgesetzt.

LC-MS (Methode 1): Rₜ = 1.68 min; MS (ESIpos): m/z = 264 (M+H)⁺

### Beispiel 6A

### 5-Fluor-1-(2-fluorbenzyl)-3-iod-1H-pyrazolo[3,4-b]pyridin

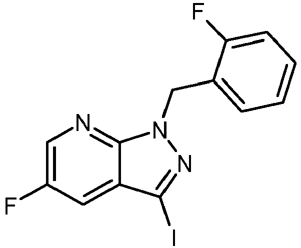

In DMF (2538 ml) wurden 141 g (462.11 mmol) der Verbindung aus Beispiel 5A vorgelegt und anschließend 96.09 g (508.32 mmol) 2-Fluorbenzylbromid sowie 165.62 g (508.32 mmol) Cäsiumcarbonat zugefügt. Die Mischung wurde zwei Stunden bei RT gerührt. Anschließend wurde die Reaktionsmischung auf gesättigte wässrige Natriumchlorid-Lösung (13670 ml) gegeben und zweimal mit Essigester (5858 ml) extrahiert. Die gesammelten organischen Phasen wurden mit gesättigter wässriger Natriumchlorid-Lösung (3905 ml) gewaschen, getrocknet, filtriert und eingeengt. Der Rückstand wurde an Kieselgel (Laufmittel: Petrolether/Essigsäureethylester 97:3) chromatographiert und die Produktfraktionen eingeengt. Der erhaltene Feststoff wurde in Dichlormethan gelöst und einmal mit gesättigter wässriger Natriumthiosulfatlösung (500 ml) und anschliessend mit gesättigter wässriger Natriumchlorid-Lösung (500 ml) gewaschen. Es wurde zur Trockene eingeengt und der Rückstand mit Diethylether aufgeschlämmt, abgesaugt und am Hochvakuum getrocknet. Man erhielt 106.6 g (62 % d. Th.) der gewünschten Verbindung.

LC-MS (Methode 1): Rₜ = 2.57 min

MS (ESIpos): m/z = 372 (M+H)⁺

¹H-NMR (400 MHz, DMSO-d₆): δ = 5.73 (s, 2H), 7.13 - 7.26 (m, 3H), 7.33 - 7.41 (m, 1H), 7.94 (dd, 1H), 8.69 - 8.73 (m, 1H).

### Beispiel 7A

### 2-[5-Fluor-1-(2-fluorbenzyl)-1H-pyrazolo[3,4-b]pyridin-3-yl]-5-nitropyrimidin-4,6-diamin

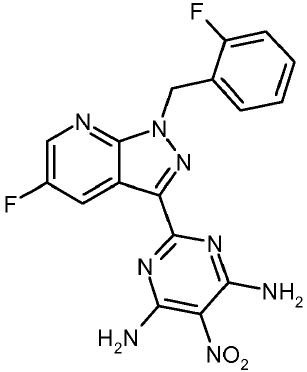

In 1,4-Dioxan (86 ml) wurden 860 mg (2.32 mmol) der Verbindung aus Beispiel 6A unter Argon vorgelegt und die Reaktionsmischung 10 min mit Argon gespült. Danach wurden 3.51 ml (6.95 mmol) Hexabutyldizinn sowie 483 mg (2.55 mmol) 2-Chlor-5-nitropyrimidin-4,6-diamin (Herstellung erfolgte analog zu: Helvetica Chimica Acta (1951), 34, 835-40) zugefügt. Anschließend wurden 860 mg (0.744 mmol) Tetrakis(triphenylphosphin)-palladium(0) zugegeben und die Reaktionsmischung über Nacht zum Rückfluss erhitzt. Danach wurde auf RT abgekühlt, mit Wasser versetzt und zweimal mit Essigsäureethylester extrahiert. Die gesammelten organischen Phasen wurden über Natriumsulfat getrocknet, filtriert und eingeengt. Der Rückstand wurde in Essigsäureethylester ausgerührt, der Feststoff abfiltriert und im Hochvakuum getrocknet. Man erhielt 355 mg (62%-ige Reinheit, 24% d. Th.) der gewünschten Verbindung. Das Rohprodukt wurde ohne weitere Reinigung umgesetzt.

LC-MS (Methode 2): Rₜ = 1.03 min

MS (ESIpos): m/z = 399 (M+H)⁺

### Beispiel 8A

### 5-Fluor-1-(2-fluorbenzyl)-1H-pyrazolo[3,4-b]pyridin-3-carbonitril

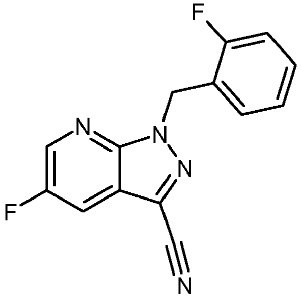

Eine Suspension aus 16.03 g (43.19 mmol) 5-Fluor-1-(2-fluorbenzyl)-3-iod-1H-pyrazolo[3,4-b]pyridin (Beispiel 6A) und 4.25 g (47.51 mmol) Kupfercyanid wurden in DMSO (120 ml) vorgelegt und 2 h bei 150°C gerührt. Nach Abkühlen wurde der Kolbeninhalt auf ca. 40°C abgekühlt, auf eine Lösung aus konz. Ammoniakwasser (90 ml) und Wasser (500 ml) gegossen, mit Essigsäureethylester (200 ml) versetzt und kurz ausgerührt. Die wässrige Phase wurde abgetrennt und noch zweimal mit Essigsäureethylester (je 200 ml) extrahiert. Die vereinigten organischen Phasen wurden zweimal mit 10%-iger wässriger Natriumchlorid-Lösung (je 100 ml) gewaschen, getrocknet und im Vakuum eingeengt. Das Rohprodukt wurde ohne weitere Reinigung umgesetzt.

Ausbeute: 11.1 g (91 % d. Theorie)

¹H-NMR (400 MHz, DMSO-d₆): δ = 5.87 (s, 2H), 7.17 - 7.42 (m, 4H), 8.52 (dd, 1H), 8.87 (dd, 1H).

### Beispiel 9A

### 5-Fluor-1-(2-fluorbenzyl)-1H-pyrazolo[3,4-b]pyridin-3-carboximidamid-Acetat

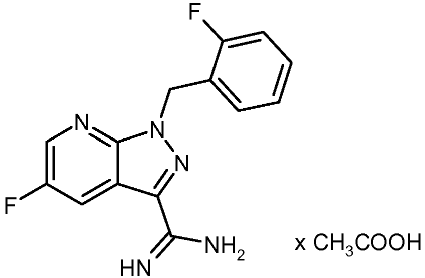

Zu 2.22 g (41.07 mmol) Natriummethanolat in Methanol (270 ml) wurden 11.1 g (41.07 mmol) 5-Fluor-1-(2-fluorbenzyl)-1H-pyrazolo[3,4-b]pyridin-3-carbonitril (Beispiel 8A) gegeben und 2 h bei RT gerührt. Danach wurden 2.64 g (49.29 mmol) Ammoniumchlorid und Essigsäure (9.17 ml) zugegeben und über Nacht zum Rückfluss erhitzt. Danach wurde bis zur Trockene eingeengt und der Rückstand in Wasser (100 ml) und Essigsäureethylester (100 ml) aufgenommen und mit 2N Ntronlauge auf pH 10 gestellt. Es wurde für ca. 1 h bei RT intensiv gerührt. Die erhaltene Suspension wurde abgesaugt und mit Essigsäureethylester (100 ml), Wasser (100 ml) und nochmals Essigsäureethylester (100 ml) nachgewaschen. Der Rückstand wurde über Phosphorpentoxid im Hochvakuum getrocknet.

Ausbeute: 9.6 g (78 % d. Th.)

MS (ESIpos): m/z = 288 (M+H)⁺

¹H-NMR (400 MHz, DMSO-d₆): δ = 1.85 (s, 3H), 5.80 (s, 2H), 7.14 - 7.25 (m, 3H), 7.36 (m, 1H), 8.42 (dd, 1H), 8.72 (dd, 1H).

### Beispiel 10A

### 2-[5-Fluor-1-(2-fluorbenzyl)-1H-pyrazolo[3,4-b]pyridin-3-yl]-5-[(E)-phenyldiazenyl]pyrimidin-4,6-diamin

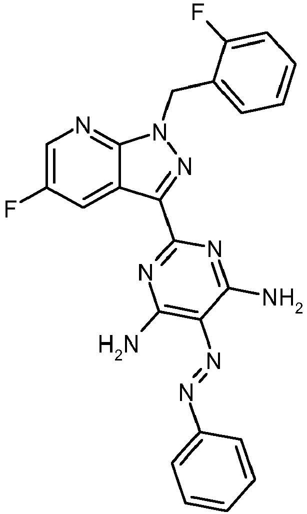

Zu Wasser (40 ml) und konz. Salzsäure (7.07 ml) wurden unter Rühren 3.85 g (41.34 mmol) Anilin gegeben und auf 0°C gekühlt. Anschliessend wurde eine Lösung von 2.85 g (41.34 mmol) Natriumnitrit in Wasser (21 ml) zwischen 0°C und 5°C zugetropft und 15 min bei 0°C nachgerührt. Danach wurde bei 0°C eine Lösung aus 4.28 g (52.25 mmol) Natriumacetat in Wasser (19 ml) zügig zugetropft und dann unter gutem Rühren eine Lösung aus 2.73 g (41.34 mmol) Malonsäuredinitril in Ethanol (10 ml) zugetropft. Nach 2 h bei 0°C wurde der entstandene Niederschlag abgesaugt und dreimal mit Wasser (je 50 ml) und mit Petrolether (50 ml) gewaschen. Der noch feuchte Rückstand wurde in DMF (46 ml) gelöst und in eine Lösung aus 9.5 g (33.07 mmol) 5-Fluor-1-(2-fluorbenzyl)-1H-pyrazolo[3,4-b]pyridin-3-carboximidamid-Acetat (Beispiel 9A) in DMF (46 ml) und Triethylamin (5.76 ml) bei genau 85°C zugetropft. Anschließend wurde 4 h bei 100°C nachgerührt und über Nacht auf RT abkühlen lassen. Die Mischung wurde auf Wasser (480 ml) gegossen und 1 h bei RT ausgerührt. Nach Absaugen des Niederschlags wurde dieser zweimal mit Wasser (je 100 ml) und zweimal mit Methanol (je 50 ml) nachgewaschen, und anschliessend im Hochvakuum getrocknet.

Ausbeute: 9.6 g (59 % d. Theorie)

LC-MS (Methode 2): Rₜ = 1.21 min

MS (ESIpos): m/z = 458 (M+H)⁺

### Beispiel 11A

### 2-[5-Fluor-1-(2-fluorbenzyl)-1H-pyrazolo[3,4-b]pyridin-3-yl]pyrimidin-4,5,6-triamin

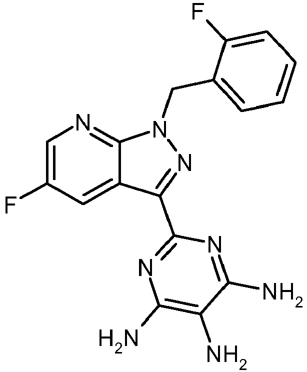

### Variante A: Darstellung ausgehend von Beispiel 7A:

In Pyridin (30 mL) wurden 378 mg (0.949 mmol) der Verbindung aus Beispiel 7A vorgelegt und anschließend 143 mg (0.135 mmol) Palladium (10%ig auf Kohle) zugefügt. Die Mischung wurde über Nacht bei RT unter Wasserstoff-Normaldruck hydriert. Anschließend wurde die Suspension durch Kieselgur filtriert und der Filterkuchen mit Ethanol nachgewaschen. Das Filtrat wurde eingeengt und lieferte 233 mg (81%ig, 51% d. Th.) der gewünschten Verbindung, die ohne weitere Reinigung umgesetzt wurden.

### Variante B: Darstellung ausgehend von Beispiel 10A:

In DMF (800 ml) wurden 39.23 g (85.75 mmol) der Verbindung aus Beispiel 10A vorgelegt und anschliessend 4 g Palladium (10%ig auf Kohle) zugefügt. Es wurde unter Rühren über Nacht bei Wasserstoff-Normaldruck hydriert. Der Ansatz wurde über Kieselgur filtriert und mit wenig DMF und danach mit wenig Methanol nachgewaschen und zur Trockene eingeengt. Der Rückstand wurde mit Essigsäureethylester versetzt und kräftig gerührt, der Niederschlag abgesaugt, mit Essigsäureethylester und Diisopropylether nachgewaschen und über Sicapent im Hochvakuum getrocknet.

Ausbeute: 31.7 g (100 % d. Theorie)

LC-MS (Methode 2): Rt = 0.81 min

MS (ESIpos): m/z = 369 (M+H)⁺

### Ausführungsbeispiele:

### Beispiel 1

### Methyl-{4,6-diamino-2-[5-fluor-1-(2-fluorbenzyl)-1H-pyrazolo[3,4-b]pyridin-3-yl]pyrimidin-5-yl}carbamat

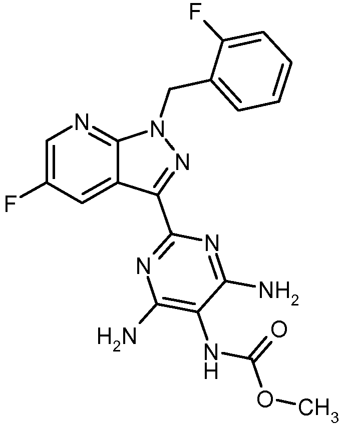

In Pyridin (600 ml) wurden 31.75 g (86.20 mmol) der Verbindung aus Beispiel 11A unter Argon vorgelegt und auf 0°C abgekühlt. Anschließend wurde eine Lösung von 6.66 ml (86.20 mmol) Chlorameisensäuremethylester in Dichlormethan (10 ml) zugetropft und die Mischung 1h bei 0°C gerührt. Danach wurde die Reaktionsmischung auf RT gebracht, im Vakuum eingeengt und mehrfach mit Toluol kodestilliert. Der Rückstand wurde mit Wasser/Ethanol verrührt und danach über eine Fritte abgesaugt und nachfolgend mit Ethanol und Essigsäureethylester gewaschen. Anschliessend wurde der Rückstand abermals mit Diethylether verrührt, abgesaugt und anschliessend im Hochvakuum getrocknet.

Ausbeute: 24.24 g (65 % d. Theorie)

LC-MS (Methode 2): Rₜ = 0.79 min

MS (ESIpos): m/z = 427 (M+H)+

¹H-NMR (400 MHz, DMSO-d₆): δ = 3.62 (br. s, 3H), 5.79 (s, 2H), 6.22 (br. s, 4H), 7.10 - 7.19 (m, 2H), 7.19 - 7.26 (m, 1H), 7.32 - 7.40 (m, 1H), 7.67 (br. s, 0.2H), 7.99 (br. s, 0.8H), 8.66 (m, 1H), 8.89 (d, 1H).

### Beispiel 2

### Methyl-{4,6-diamino-2-[5-fluor-1-(2-fluorbenzyl)-1H-pyrazolo[3,4-b]pyridin-3-yl]pyrimidin-5-yl}methylcarbamat

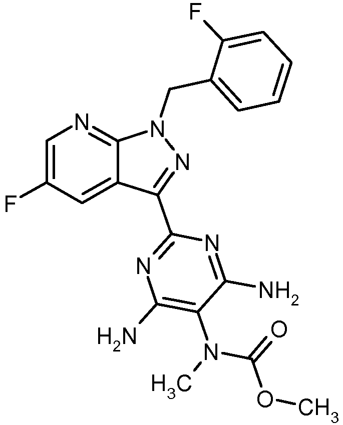

200 mg (0.469 mmol) Methyl-{4,6-diamino-2-[5-fluor-1-(2-fluorbenzyl)-1H-pyrazolo[3,4-b]pyridin-3-yl]pyrimidin-5-yl}carbamat (Beispiel 1) wurden in THF (5ml) bei 0°C vorgelegt. Danach wurden 0.704 ml (0.704 mmol) Lithiumhexamethyldisilazan-Lösung (1M in THF) zugegeben und 20 min bei dieser Temperatur gerührt. Anschliessend wurden 43.8 µl (0.704 mmol) Iodmethan zugegeben und auf RT erwärmt. Nach 1h bei dieser Temperatur wurde mit Wasser abgebrochen (1 ml), eingeengt und der Rückstand mittels präparativer RP-HPLC (Wasser (+0.05 % Ameisensäure)-Acetonitril Gradient) getrennt.

Ausbeute: 90 mg (44 % d. Theorie)

LC-MS (Methode 2): Rₜ = 0.85 min

MS (ESIpos): m/z = 441 (M+H)⁺

¹H-NMR (400 MHz, DMSO-d₆): δ = 3.00 (s, 3H), 3.53 (s, 2.2H), 3.66 (s, 0.8H), 5.81 (s, 2H), 6.57 (br. s, 4H), 7.13 (m, 2H), 7.22 (m, 1H), 7.35 (m, 1H), 8.67 (m, 1H), 8.87 (dd, 1H).

### Beispiel 3

### Methyl-{4,6-diamino-2-[5-fluor-1-(2-fluorbenzyl)-1H-pyrazolo[3,4-b]pyridin-3-yl]pyrimidin-5-yl}(2,2,2-trifluorethyl)carbamat

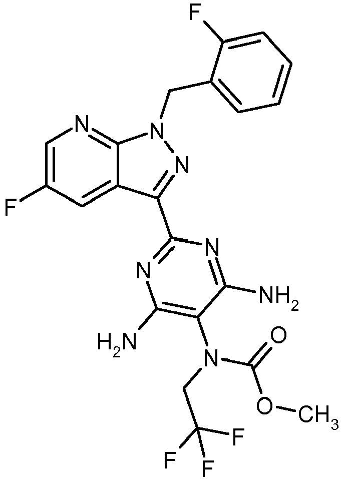

Es wurden 3.470 g (8.138 mmol) der Verbindung aus Beispiel 1 in 35 ml THF suspendiert, bei 0°C mit 358 mg (8.952 mmol) Natriumhydrid (60%-ige Suspension in Mineralöl) versetzt und 90 Min. bei 0°C gerührt, wobei sich eine Lösung bildete. Es wurden 2.519 g (8.952 mmol) 2,2,2-Trifluorethyltrichlormethansulfonat addiert und das Gemisch für 48 h bei RT gerührt. Anschliessend wurde mit Wasser verrührt und am Rotationsverdampfer eingeengt. Der Rückstand wurde in Essigsäureethylester aufgenommen, die organische Phase zweimal mit Wasser gewaschen und über Natriumsulfat getrocknet. Es wurden 5.005 g der Zielverbindung erhalten (79 % d. Th., Reinheit nach HPLC 65%). 250 mg des Rückstands wurden mittels präparativer HPLC gereinigt (Eluent: Methanol/Wasser, Gradient 30:70 → 90:10).

LC-MS (Methode 2): Rₜ = 0.97 min; MS (EIpos): m/z = 509 [M+H]⁺.

¹H-NMR (400 MHz, DMSO-d₆): δ [ppm] = 3.63 (s, 3H), 4.06-4.15 (m, 2H), 5.80 (s, 2H), 6.46 (s br, 4H) 7.11-7.15 (m, 2H), 7.20-7.25 (m, 1H), 7.33-7.38 (m, 1H), 8.66 (dd, 1H), 8.91 (dd, 1H).

### B) Bewertung der physiologischen Wirksamkeit

Die Eignung der erfindungsgemäßen Verbindungen zur Behandlung von Erkrankungen mit erhöhtem Hb Konzentrationen wie kann in folgenden Assaysystemen gezeigt werden:

### 1.) In vitro Assays

### 1.a.) sGC Enzym Assay: Stimulation der rekombinanten löslichen Guanylatcyclase (sGC) in vitro

Die Untersuchungen zur Stimulation der rekombinanten löslichen Guanylatcyclase (sGC) durch die erfindungsgemäßen Verbindungen mit und ohne Natriumnitroprussid sowie mit und ohne den Häm-abhängigen sGC-Inhibitor 1*H-*1,2,4-Oxadiazolo[4,3-a]chinoxalin-1-on (ODQ) werden nach der in folgender Literaturstelle im Detail beschriebenen Methode durchgeführt: M. Hoenicka, E.M. Becker, H. Apeler, T. Sirichoke, H. Schroeder, R. Gerzer und J.-P. Stasch, "Purified soluble guanylyl cyclase expressed in a baculovirus/Sf9 system: Stimulation by YC-1, nitric oxide, and carbon oxide", J. Mol. Med. 77 (1999), 14-23. Die Häm-freie Guanylatcyclase wird durch Zugabe von Tween 20 zum Probenpuffer (0.5% in der Endkonzentration) erhalten. Die Aktivierung der sGC durch eine Prüfsubstanz wird als x-fache Stimulation der Basalaktivität angegeben. Lösliche Guanylatcyclase (sGC) setzt unter Stimulation GTP zu cGMP und Pyrophosphat (PPi) um. PPi wird mit Hilfe des nachfolgend beschriebenen Tests nachgewiesen. Das im Test entstehende Signal nimmt mit fortschreitender Umsetzung zu und dient als Maß für die sGC-Enzymaktivität unter der gegebenen Stimulation.

Zur Durchführung des Tests werden 29 µl Enzymlösung [0-10 nM lösliche Guanylatcyclase (hergestellt nach Hönicka et al., J. Mol. Med. 77, 14-23 (1999)) in 50 mM TEA, 2 mM MgCl₂, 0.1% BSA (Fraktion V), 0.005% Brij^{®}, pH 7.5] in eine Mikroplatte vorgelegt und 1 µl der zu testenden Substanz (als seriell verdünnte Lösung in DMSO) hinzugegeben. Der Ansatz wird 10 min bei Raumtemperatur inkubiert. Anschließend werden 20 µl Detektionsmix [1.2 nM Firefly-Luciferase (*Photinus pyralis*-Luciferase, Fa. Promega), 29 µM Dehydro-Luziferin (hergestellt nach Bitler & McElroy, Arch. Biochem. Biophys. 72, 358 (1957)), 122 µM Luziferin (Fa. Promega), 153 µM ATP (Fa. Sigma) und 0.4 mM DTT (Fa. Sigma) in 50 mM TEA, 2 mM MgCl₂, 0.1% BSA (Fraktion V), 0.005% Brij^{®}, pH 7.5] zugegeben. Die Enzymreaktion wird durch Zugabe von 20 µl Substratlösung [1.25 mM Guanosin-5'-triphosphat (Fa. Sigma) in 50 mM TEA, 2 mM MgCl₂, 0.1% BSA (Fraktion V), 0.005% Brij^{®}, pH 7.5] gestartet und kontinuierlich luminometrisch vermessen. Das Maß der Stimulation durch die zu testende Substanz kann relativ zum Signal der nicht stimulierten Reaktion bestimmt werden.

Durch Zugabe von 25 µM 1*H*-1,2,4-Oxadiazolo[4,3-a]chinoxalin-1-on (ODQ) zur Enzymlösung und anschließende 30-minütige Inkubation wird die Aktivierung der Häm-freien Guanylatcyclase untersucht und mit der Stimulation des nativen Enzyms verglichen.

### 1.b. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

### 1.c Gefäßrelaxierende Wirkung in vitro

Kaninchen werden durch Nackenschlag betäubt und entblutet. Die Aorta wird entnommen, von anhaftendem Gewebe befreit, in 1.5 mm breite Ringe geteilt und einzeln unter einer Vorspannung in 5 ml-Organbäder mit 37°C warmer, Carbogen-begaster Krebs-Henseleit-Lösung folgender Zusammensetzung gebracht (jeweils mM): Natriumchlorid: 119; Kaliumchlorid: 4.8; Calciumchlorid-Dihydrat: 1; Magnesiumsulfat-Heptahydrat: 1.4; Kaliumdihydrogenphosphat: 1.2; Natriumhydrogencarbonat: 25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung zugesetzt und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50% zu reduzieren (IC₅₀-Wert). Das Standardapplikationsvolumen beträgt 5 µl, der DMSO-Anteil in der Badlösung entspricht 0.1%.

### 2.) in vivo Messung

### 2.a). Radiotelemetrische Blutdruckmessung an wachen, spontan hypertensiven Ratten

Für die im Folgenden beschriebene Blutdruckmessung an wachen Ratten wird ein im Handel erhältliches Telemetriesystem der Firma DATA SCIENCES INTERNATIONAL DSI, USA eingesetzt.

Das System besteht aus 3 Hauptkomponenten:
- Implantierbare Sender (Physiotel® Telemetrietransmitter)
- Empfänger (Physiotel® Receiver), die über einen Multiplexer (DSI Data Exchange Matrix) mit einem
- Datenakquisitionscomputer
verbunden sind.

Die Telemetrieanlage ermöglicht eine kontinuierliche Erfassung von Blutdruck Herzfrequenz und Körperbewegung an wachen Tieren in ihrem gewohnten Lebensraum.

### Tiermaterial

Die Untersuchungen werden an ausgewachsenen weiblichen spontan hypertensiven Ratten (SHR Okamoto) mit einem Körpergewicht von >200 g durchgeführt. SHR/NCrl von Okamoto Kyoto School of Medicine, 1963 wurden aus männlichen Wistar Kyoto Ratten mit stark erhöhtem Blutdruck und weiblichen mit leicht erhöhtem Blutdruck gekreuzt und in der F13 an die U.S. National Institutes of Health abgegeben.

Die Versuchstiere werden nach Senderimplantation einzeln in Makrolon - Käfigen Typ 3 gehalten. Sie haben freien Zugang zu Standardfutter und Wasser.

Der Tag - Nacht - Rhythmus im Versuchslabor wird per Raumbeleuchtung um 6:00 Uhr morgens und um 19:00 Uhr abends gewechselt.

### Senderimplantation

Die eingesetzten Telemetriesender TA11 PA - C40 werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobabital (Nembutal, Sanofi: 50mg/kg i.p.) narkotisiert und an der Bauchseite weiträumig rasiert und desinfiziert. Nach Eröffnung des Bauchraumes entlang der Linea alba wird der flüssigkeitsgefüllte Meßkatheter des Systems oberhalb der Bifurcation nach cranial in die Aorta descendens eingesetzt und mit Gewebekleber (VetBonD TM, 3M) befestigt. Das Sendergehäuse wird intraperitoneal an der Bauchwandmuskulatur fixiert und die Wunde wird schichtweise verschlossen.

Postoperativ wird zur Infektionsprophylaxe ein Antibiotikum verabreicht (Tardomyocel COMP Bayer 1ml/kg s.c.)

### Substanzen und Lösungen

Wenn nicht anders beschrieben werden die zu untersuchenden Substanzen jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5%-iger Tylose suspendiert.

Eine Lösungsmittel- behandelte Gruppe von Tieren wird als Kontrolle eingesetzt.

### Versuchsablauf

Die vorhandene Telemetrie - Meßeinrichtung ist für 24 Tiere konfiguriert. Jeder Versuch wird unter einer Versuchsnummer registiert (VJahr Monat Tag).

Den in der Anlage lebenden instrumentierten Ratten ist jeweils eine eigene Empfangsantenne zugeordnet (1010 Receiver, DSI).

Die implantierten Sender sind über einen eingebauten Magnetschalter von außen aktivierbar. Sie werden bei Versuchsvorlauf auf Sendung geschaltet. Die ausgestrahlten Signale können durch ein Datenakquisitionssystem (Dataquest TM A.R.T. for WINDOWS, DSI) online erfasst und entsprechend aufgearbeitet werden. Die Ablage der Daten erfolgt jeweils in einem hierfür eröffneten Ordner der die Versuchsnummer trägt.

Im Standardablauf werden über je 10 Sekunden Dauer gemessen:
- Systolischer Blutdruck (SBP)
- Diastolischer Blutdruck (DBP)
- Arterieller Mitteldruck (MAP)
- Herzfrequenz (HR)
- Aktivität (ACT).

Die Messwerterfassung wird rechnergesteuert in 5 Minuten Abständen wiederholt. Die als Absolutwert erhobenen Quelldaten werden im Diagramm mit dem aktuell gemessenen Barometerdruck (Ambient Pressure Reference Monitor; APR-1) korrigiert und in Einzeldaten abgelegt. Weitere technische Details sind der umfangreichen Dokumentation der Herstellerfirma (DSI) zu entnehmen.

Wenn nicht anders beschrieben erfolgt die Verabreichung der Prüfsubstanzen am Versuchstag um 9.00 Uhr. Im Anschluss an die Applikation werden die oben beschriebenen Parameter 24 Stunden gemessen.

### Auswertung

Nach Versuchsende werden die erhobenen Einzeldaten mit der Analysis-Software (DATAQUEST TM A. R.T. TM ANALYSIS) sortiert. Als Leerwert werden hier 2 Stunden vor Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten Average) und als Textdatei auf einen Datenträger übertragen. Die so vorsortierten und komprimierten Messwerte werden in Excel-Vorlagen übertragen und tabellarisch dargestellt. Die Ablage der erhobenen Daten erfolgt pro Versuchstag in einem eigenen Ordner, der die Versuchsnummer trägt. Ergebnisse und Versuchsprotokolle werden in Papierform nach Nummern sortiert in Ordnern abgelegt.

### Literatur

Klaus Witte, Kai Hu, Johanna Swiatek, Claudia Müssig, Georg Ertl and Björn Lemmer: Experimental heart failure in rats: effects on cardiovascular circadian rhythms and on myocardial β-adrenergic signaling. Cardiovasc Res 47 (2): 203-405, 2000; Kozo Okamoto: Spontaneous hypertension in rats. Int Rev Exp Pathol 7: 227- 270, 1969; Maarten van den Buuse: Circadian Rhythms of Blood Pressure, Heart Rate, and Locomotor Activity in Spontaneously Hypertensive Rats as Measured With Radio-Telemetry. Physiology & Behavior 55(4): 783-787, 1994

### 2.b.) Messung von Blutfluss und Blutdruck in Ratten

250 - 350 g schwere Wistar Ratten (Hsd Cpb:Wu) bzw. 330 - 520 g schwere ZDF Ratten (ZDF/Crl-Lepr fa/fa) werden mittels 2.5% Isofluran im Sauerstoff - Lachgasgemisch (40:60) narkotisiert. Zur Bestimmung des Blutflusses in der A. carotis, A. femoralis wird die narkotisierte Ratte in Rückenlage gebracht und anschließend die linkseitige A. carotis und rechtsseitige A. femoralis vorsichtig freipräpariert. Der Blutfluss wird durch das Anbringen von Flusssonden (Transonic Flowprobe) an den Gefäßen gemessen. Durch das Einbringen eines PE50-Arterien-Katheter in die linksseitige A. femoralis wird der Blutdruck und die Herzratte (Transducer Ref. 5203660: Fa.Braun CH) bestimmt. Die Substanzgabe erfolgt als Bolus bzw. Dauerinfusion über einen Venen-Katheder in der linksseitigen V. femoralis.

### 2.c.) Prüfung von durchblutungsfördernden Substanzen (Mikrozirkulation)

Zur Erzeugung einer Minderperfusion wird bei Ratten (z.B. ZDF Ratten) in Narkose (z.B. Inhalationsnarkose Isofluran, Enfluran) unter sterilen Bedingungen die rechte A. iliaca externa ligiert. Je nach Kollateralisierungsgrad der Tiere muß zur Erzeugung einer Minderperfusion zusätzlich noch die A. femoralis ligiert werden. Im Anschluß an die Operation oder auch präventiv werden die Versuchstiere oral, intragastral (Magensonde, Futter- oder Trinkwasseraufnahme), intraperitoneal, intravenös, intraarteriell, intramuskulär, inhalativ oder subcutan mit den Prüfsubstanzen behandelt. Alternativ wird bei den Tieren extrazelluläres Hämoglobin appliziert, das zuvor aus Spendertieren gewonnen wurde. Die Prüfsubstanzen werden akut oder für bis zu 5 Wochen einmal oder mehrfach enteral oder parenteral täglich appliziert oder es erfolgt eine Dauerapplikation über subkutan implantierte osmotische Minipumpen (z.B. Alzet Pumpen). Die Mikroperfusion und Temperatur der unteren Extremitäten werden während dem Versuch dokumentiert. Dabei wird den Ratten unter Narkose eine temperatursensitive Laserdopplersonde (Periflux) auf die Pfoten geklebt und somit Microperfusion und Hauttemperatur gemessen. Je nach Versuchsplan werden Proben wie Blut (Interimsdiagnostik) und sonstige Körperflüssigkeiten oder Organe entnommen, um in vitro weitere Untersuchungen durchzuführen oder es wird Zur Dokumentation der Hämodynamik über einen Katheter in der A. carotis Blutdruck und Herzfrequenz gemessen. Am Ende des Experiments werden die Tiere schmerzlos getötet.

### 2.d.) Prüfung von durchblutungsfördernden Substanzen (Motorische Funktion) im Laufradversuch

Zur Bestimmung der motorischen Funktion wird das Laufverhalten von Mäusen (z.B. eNOS knock out Mäuse, Wildtyp Mäuse C-57 B16 oder ApoE knock out Mäuse, Sichelzellmäuse, ...) in Laufrädern untersucht. Um die Mäuse an die freiwillige Benutzung des Laufrades zu gewöhnen, werden 4-5 Wochen vor Beginn des Versuches die Tiere in Käfigen mit Laufrad vereinzelt und trainiert. 2 Wochen vor Start des Experimentes werden die Bewegungen der Mäuse im Laufrad durch eine Photozelle mittels Computer aufgezeichnet und verschiedene Laufparameter wie z.B. die tägliche Laufstrecke, die zurückgelegten Einzelstrecken, aber auch Ihre zeitliche Verteilung über den Tag bestimmt. Die Tiere werden nach ihrem natürlichen Laufverhalten in Gruppen (8-12 Tiere) randomisiert (Kontrollgruppe, Sham Gruppe und ein bis mehrere Substanzgruppen). Im Anschluß daran werden die Versuchstiere oral, intragastral (Magensonde, Futter- oder Trinkwasseraufnahme), intraperitoneal, intravenös, intraarteriell, intramuskulär, inhalativ oder subcutan mit den Prüfsubstanzen behandelt. Die Prüfsubstanzen werden für bis zu 5 Wochen einmal oder mehrfach enteral oder parenteral täglich appliziert oder es erfolgt eine Dauerapplikation über subkutan implantierte osmotische Minipumpen. Das Laufverhalten der Tiere wird über 5-8 Wochen beobachtet und aufgezeichnet. Am Ende des Experiments werden die Tiere schmerzlos getötet. Je nach Versuchsplan werden Proben wie Blut und sonstige Körperflüssigkeiten oder Organe entnommen, um in vitro weitere Untersuchungen durchzuführen (S. Vogelsberger Neue Tiermodelle für die Indikation Claudicatio Intermittens (Taschenbuch), Verlag: VVB Laufersweiler Verlag (März 2006), ISBN-10: 383595007X, ISBN-13: 978-3835950078).

### 2.e.) Hämodynamik im narkotisierten Hund

Es werden 25-35 kg schwere gesunde oder herzinsuffiziente Mongrel^{®} Hunde (Marshall BioResources, Marshall Farms Inc; Clyde NY; USA) beiderlei Geschlechts verwendet. Die Narkose wird eingeleitet durch langsame i.v. Gabe von 25 mg/kg Natrium Thiopental (Trapanal^{®}) und 0.15 mg/kg Alcuronium Chlorid (Alloferin^{®}) und während des Experimentes aufrechterhalten mittels einer Dauerinfusion aus 0.04 mg/kg*h Fentanyl (Fentanyl^{®}), 0.25 mg/kg*h Droperidol (Dihydrobenzperidol^{®}) und 15 µg/kg/h Alcuronium Chloride (Alloferin^{®}). Nach der Intubation werden die Tiere über die Beatmungsmaschine mit konstanten Atemvolumen beatmet, so dass eine endtidale CO₂ Konzentration von etwa 5% erreicht wird. Die Beatmung erfolgt mit Raumluft, angereichert mit ca. 30% Sauerstoff (Normoxie). Zur Messung der hämodynamischen Parameter wird ein mit Flüssigkeit gefüllter Katheter in die A. femoralis zur Messung des Blutdruckes implantiert. Ein 2-lumiger Swan-Ganz^{®} Katheter wird über die V. jugularis in die Pulmonalarterie eingeschwemmt (distales Lumen zur Messung des pulmonalarteriellen Druckes, proximales Lumen zur Messung des zentralen Venendruckes). Mittels Temperaturfühler an der Spitze des Katheters wird der kontinuierliche Cardiac Output (CCO) bestimmt. Der Blutfluss wird an unterschiedlichen Gefäßbetten wie z.B. der Koronararterie, der A. Carotis oder der Femoralarterie durch das Anbringen von Flusssonden (Transonic Flowprobe) an den jeweiligen Gefäßen gemessen. Der linksventrikuläre Druck wird nach Einführung eines Mikro-Tip-Katheters (Millar^{®} Instruments) über die A. carotis in den linken Ventrikel gemessen und davon das dP/dt als Maß für die Kontraktilität abgeleitet. Substanzen werden i.v. über die V. femoralis oder intraduodenal als kumulative Dosis-Wirkungskurve (Bolus oder Dauerinfusion) appliziert. Die hämodynamischen Signale werden mittels Druckaufnehmern / Verstärkern und PONEMAH^{®} als Datenerfassungssoftware aufgezeichnet und ausgewertet.

Zur Induktion von Herzinsuffizienz wird den Hunden unter sterilen Bedingungen ein Schrittmacher implantiert. Nach Induktion der Narkose mittels Pentobarbital-Na (15 to 30 mg kg⁻¹ i.v.) gefolgt von einer Intubation und anschliessender Ventilation (room air; Sulla 808, Dräger^{®}, Germany) wird die Narkose durch kontinuierliche Infusion von Pentobarbital (1-5 mg kg⁻¹ h⁻¹) und Fentanyl (10-40 µg kg⁻¹ h⁻¹) aufrechterhalten. Ein Schrittmacherkabel (Setrox S60^{®}, Biotronik, Germany) wird implantiert über eine Insertion der linken Vena Jugularis und im rechten Ventrikel plaziert. Das Kabel wird mit dem Schrittmacher(Logos^{®}, Biotronik, Germany) verbunden, der in einer kleinen subkutanen Tasche zwischen den Schulterblättern positioniert wird. Erst 7 Tage nach dem Eingriff wird das ventrikuläre Pacing zur Erzielung einer Herzinsuffiziens bei einer Frequenz von 220 Schlägen/minüber einen Zeitraum von 10-28 Tagen gestartet.

### 2.f.) Hämodynamik im narkotisierten Ferkel

Hemodynamic parameters were continuously measured by intravascular catheters connected by Combitrans® transducers to Gould® transducers (series 6600) connected to the PoNeMah® acquisition and analysis system.

Es werden 2-6 kg schwere gesunde Göttinger Minipigs^{®} Ellegaard (Ellegaard, Denmark) beiderlei Geschlechts verwendet. Die Narkose erfolgt durch die Gabe von 7.5 mg/ml Ketamin, 1.125 mg/ml Midazolam (Infusionsrate 6 - 25 ml/h) und 0.6 mg/h Pancuroniumbromid (Pancuronium®, Organon, Oss, Netherland). Nach der Intubation werden die Tiere über die Beatmungsmaschine mit konstanten Atemvolumen beatmet (10-12 ml/kg, 35 Atemzüge/min Avea, Viasys Healthcare, USA), so dass eine endtidale CO₂ Konzentration von etwa 5% erreicht wird. Die Beatmung erfolgt mit Raumluft, angereichert mit ca. 40% Sauerstoff (Normoxie). Zur Messung der hämodynamischen Parameter werden Katheter in die A. femoralis zur Messung des Blutdruckes und ein Swan-Ganz^{®} Katheter über die V. jugularis in die Pulmonalarterie eingeschwemmt (distales Lumen zur Messung des pulmonalarteriellen Druckes, proximales Lumen zur Messung des zentralen Venendruckes). Mittels Temperaturfühler an der Spitze des Katheters wird der kontinuierliche Cardiac Output (CCO) bestimmt. Die hämodynamischen Signale werden mittels Druckaufnehmern / Verstärkern und PONEMAH^{®} als Datenerfassungssoftware aufgezeichnet und ausgewertet.

### 2.g.) Inhalation von sGC Stimulatoren und Aktivatoren

Die Experimente wurden in narkotisierten Göttinger Minischweinen (2.5-4 kg, Narkose: 7.5 mg/ml Ketamin, 1.125 mg/ml Midazolam (Infusionsrate 6 - 25 ml/h) and 0.6 mg/h Pancuroniumbromid (Pancuronium®, Organon, Oss, Niederlande), narkotisierten Ratten und wachen telemetrisch instrumentierten Hunden durchgeführt. Die narkotisierten Tiere wurden nach Intubation künstlich mit Raumluft, angereichert mit 40% Sauerstoff beatmet (Schweine: 10-12 ml/kg, 35 breaths/min using Avea, Viasys Healthcare, USA). Akute Pulmonale Hypertonie wurde durch eine Thromboxan A2 Analoga Infusion (0.12 - 0.14 µg/kg/min 9,11-dideoxy-9α,11α-epoxymethanoprostaglandin F2α (U-46619, Sigma, Germany)) oder Monocrotalingabe bei ratten induziert. Die Vernebelung der Substanzen erfolgte unter Verwendung des Nebutec® oder Aeroneb® Pro Vernebler Systems oder nach Feststoffverneblung (Pauluhn), die in den Inspirationsschenkel der Beatmung geschaltet wurden. Die Substanzen wurden als Feststoffe oder Lösungen in Abhängigkeit zur Molekularstruktur eingesetzt. Die hämodynamischen Signale werden mittels Druckaufnehmern / Verstärkern und PONEMAH^{®} oder CardioMems^{®} als Datenerfassungssoftware aufgezeichnet und ausgewertet.

### 2.h.) Testung der Schmerzschwelle/Latenzzeit im Hot Plate Test

Hot Plate: Typ Socrel DS 37, Firma Ugo Basile.Die Messung wird an Mäusen (z.B. NMRI Maüse, Sichelzellmäuse) durchgefürt. Die Temperatur der Hot Plate beträgt 50°C , die maximale Dauer der messung je Tier 90 Sekunden (cut off Zeit). Unmittelbar vor der Applikation der Testsubstanz (Prüfsubstanzen z.B. über Futter, Trinkwasser, po Gabe, Alzet Pumpen über mehrere Tage) wird für jedes Tier ein Hot Plate Vorwert bestimmt. Kriterien für die Latenzzeit (in sec.) bis zur Schmerzreaktion sind Pfotenschütteln, Pfotenlecken oder Springen. Nach ca. 8-tägiger Verabreichung der Testsubstanz wird unter denselben Bedingungen das 2. Messergebnis durchgeführt.

## Patentansprüche

1. Verwendung von Aktivatoren der löslichen Guanylatzyklase der Formeln (5) und (9) bis (16) 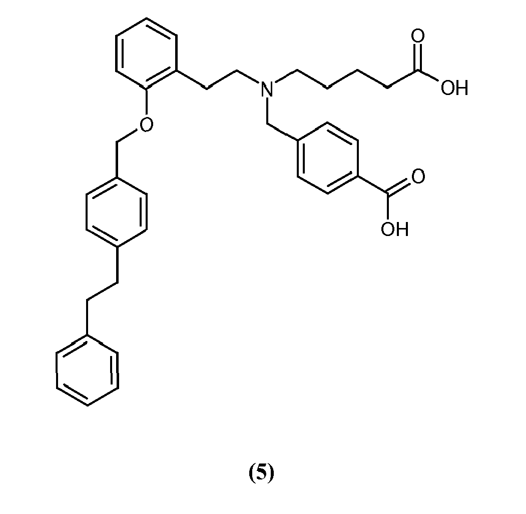 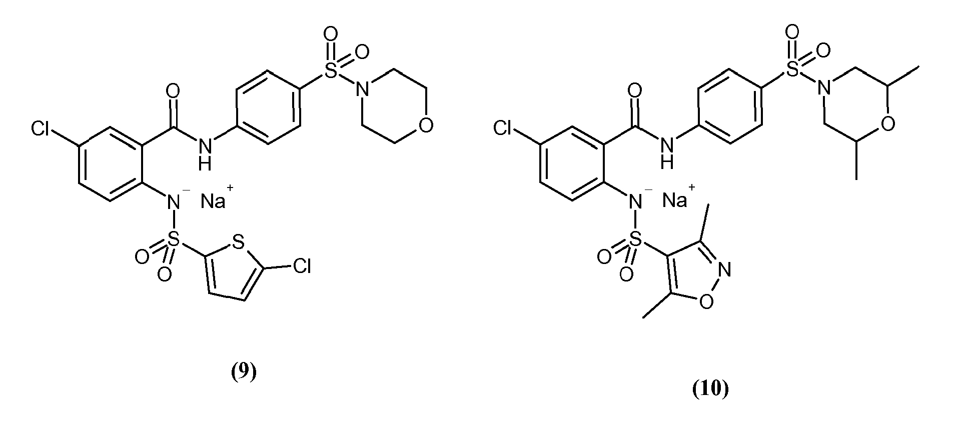 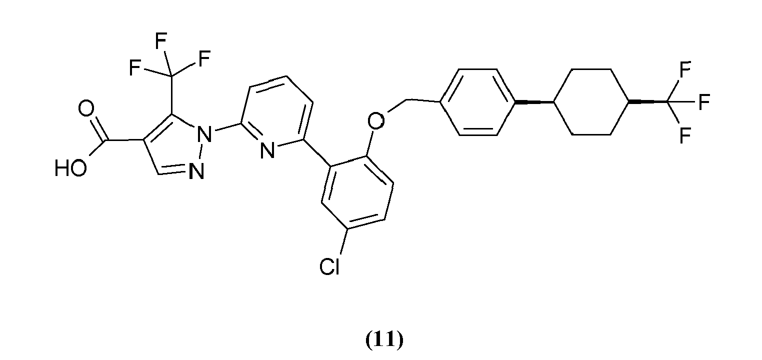 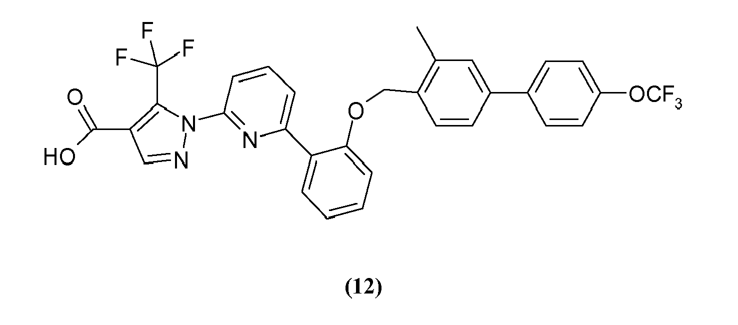 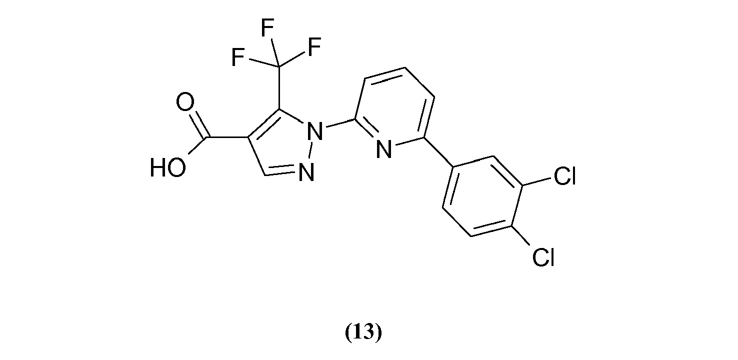 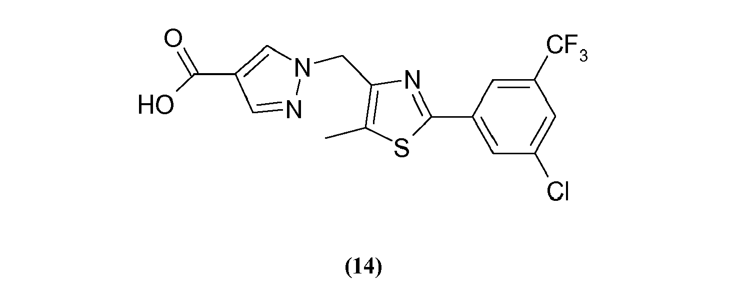 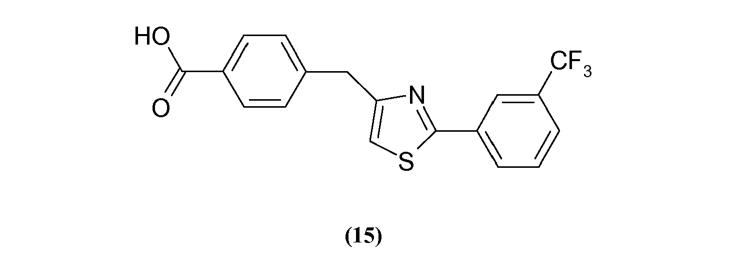 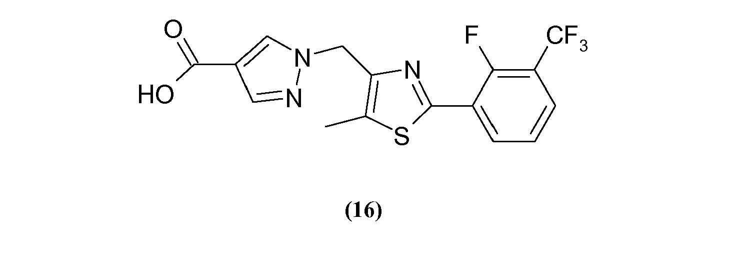 Behandlung von Sichelzellanämie und Konservierung von Blutersatzstoffen.

2. Verwendung von Verbindung der Formel 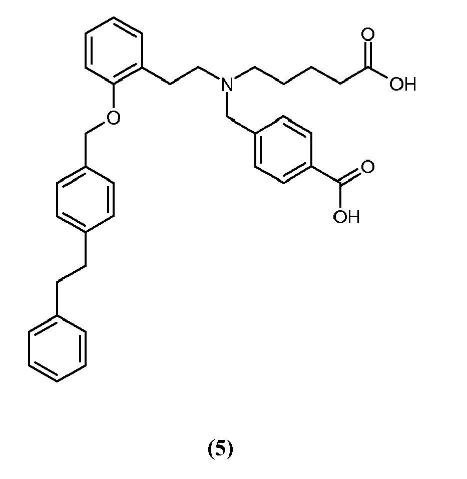 zur Behandlung und/oder Prophylaxe von Sichelzellanämie und Konservierung von Blutersatzstoffen.

3. Verwendung von einer oder mehrerer Verbindungen der Formeln (1) bis (26) gemäß Anspruch 1 zur Behandlung und/oder Prophylaxe von traumatisierten Patienten, welche einen künstlichen Blutersatzstoff erhalten.

4. Verwendung von einer oder mehrerer der erfindungsgemäßen Verbindungen gemäß der Formeln (5) und (9) bis (16) gemäß Anspruch 1 zur Herstellung eines Arzneimittels zur Behandlung und/oder Prophylaxe von Sichelzellanämie und Konservierung von Blutersatzstoffen.

5. Arzneimittel, enthaltend eine oder mehrere erfindungsgemäße Verbindung der Formeln (5) und (9) bis (16) gemäß Anspruch 1 und einen oder mehrere weitere Wirkstoffe, insbesondere zur Behandlung und/oder Prophylaxe von Sichelzellanämie und Konservierung von Blutersatzstoffen.

6. Verwendung von einer oder mehrerer Verbindungen der der Formeln (5) und (9) bis (16) gemäß Anspruch 1 in Kombination mit einem oder mehreren der folgenden PDE5 Inhibitoren:
*Tadalafil* ((6R,12aR) -2,3,6,7,12,12a - Hexahydro - 2 - methyl - 6 - (3,4-methylene - dioxyphenyl) pyrazino(1',2':1,6) pyrido(3,4-b)indole-1,4-dione), *Vardenafil* (2-(2-Ethoxy-5-(4-ethylpiperazin-1-yl-1-sulfonyl)phenyl)-5-methyl-7-propyl-3 H-imidazo (5,1-f) (1,2,4)triazin-4-one), *Sildenafil* (3-[2-ethoxy-5-(4-methylpiperazin-1-yl)sulfonyl-phenyl]- 7-methy 1- 9- propy 1-2,4,7,8- tetrazabicyclo [4.3.0]nona -3,8,10-trien-5-one), *Udenafil* 5-[2-propyloxy-5-(1-methyl-2-pyrrolidinylethylamidosulfonyl)phenyl]-methyl-3-propyl-1,6-dihydro-7H-pyrazolo(4,3-d)pyrimidine-7-one, *Dasantafil* 7-(3-Bromo-4-methoxybenzyl)-1-ethyl-8-[[(1,2)-2-hydroxycyclopentyl]amino]-3-(2-hydroxyethyl)-3,7-dihydro-1-purine-2,6-dione, *Avanafil* 4-{[(3-chloro-4-methoxyphenyl)methyl]amino}-2-[(2S)-2-(hydroxymethyl)pyrrolidin-1-yl]-N-(pyrimidin-2-ylmethyl)pyrimidine-5-carboxamide, Mirodenafil, Lodenafil, UK 369.003, UK 371.800, *SLx 2101* of Surface Logix, *LAS 34179*Triazolo[1,2-]xanthine,6-methyl-4-propyl-2-[2-propoxy-5-(4-methylpiperazino)sulfonyl]phenyl oder Salze, Hydrate oder Hydrate der Salze davon zur Behandlung und/oder Prophylaxe von Sichelzellanämie und Konservierung von Blutersatzstoffen.

7. Verwendung von einer oder mehrerer Verbidnung der Formel (5) gemäß Anspruch 1 in Kombination mit Vardenafil und/oder Sildenafil gemäß Anspruch 6 zur Behandlung und/oder Prophylaxe von Sichelzellanämie und Konservierung von Blutersatzstoffen.

8. Verwendung von einer oder mehrerer der Verbindungen der Formeln Formeln (5) und (9) bis (16) gemäß Anspruch 1 in Kombination mit PDE5 Inhibitoren gemäß Anspruch 6 zur Behandlung und/oder Prophylaxe von traumatisierten Patienten, welche einen künstlichen Blutersatzstoff erhalten.

9. Verwendung einer oder mehrerer der erfindungsgemäßen Verbindungen gemäß der Formeln Formeln (5) und (9) bis (16) gemäß Anspruch 1 in Kombination mit PDE5 Inhibitoren gemäß Anspruch 6 zur Herstellung eines Arzneimittels zur Behandlung und/oder Prophylaxe von Sichelzellanämie und Konservierung von Blutersatzstoffen.

10. Arzneimittel, enthaltend eine oder mehrere der erfindungsgemäßen Verbindungen der Formeln Formeln (5) und (9) bis (16) gemäß Anspruch 1 in Kombination mit PDE5 Inhibitoren gemäß Anspruch 6 und einen oder mehrere weitere Wirkstoffe, insbesondere zur Behandlung und/oder Prophylaxe von Sichelzellanämie und Konservierung von Blutersatzstoffen.

11. Verwendung von einer oder mehrerer Verbindungen der Formeln Formeln (5) und (9) bis (16) gemäß Anspruch 1 zur Behandlung und/oder Prophylaxe von Sichelzellanämie wobei die Anwendung inhalativ erfolgt.

12. Verwendung von einer oder mehrerer Verbindungen der Formeln Formeln (5) und (9) bis (16) gemäß Anspruch 1 in Kombination mit PDE5 Inhibitoren gemäß Anspruch 8 zur Behandlung und/oder Prophylaxe von Sichelzellanämie wobei die Anwendung inhalativ erfolgt.
